# EUROPEAN PATENT APPLICATION

(11) **EP 1 696 040 A1**
(43) Date of publication of application: **30.08.2006**
(21) Application number: 05290451.3
(22) Date of filing: 28.02.2005
(51) Int. Cl.: C12Q 1/70

(54) **Method of genotyping and phenotyping hepatitis B viruses resistant to antiviral molecules**

(71) Applicant: Eurofins Viralliance, Inc., Memphis TN 38119 (US); INSTITUT NATIONAL DE LA SANTE ET DE LA RECHERCHE MEDICALE (INSERM), 75013 Paris (FR)
(72) Inventor: Zoulim, Fabien, 69570 Dardilly (FR); Barraud, Luc, 69220 Belleville sur Saone (FR); Durantel, Sandra, 69740 Genas (FR); Lebel-Binay, Sophie, 94800 Villejuif (FR); Durantel, David, 69740 Genas (FR); Trepo, Christian, 69500 Bron (FR)
(74) Representative: Colombet, Alain André

(57) **Abstract**

The invention provides a method of preparation of HBV genomic amplicons which makes it possible, using the same amplicon obtained from a patient, to evaluate HBV strains resistant to antiviral agents by analysing, their genotype, their phenotype and their replicative capacity. The method of the invention guarantees to obtain the closest *in vitro* replication capability compared to the *in vivo* replication and ensures that the influences of all the mutations present on a viral genome are taken into account when evaluating the drug sensitivity and the replicative capacity.

## Description

The present invention relates to the field of analysis of the hepatitis B virus (HBV) for diagnostic and therapeutic purposes. More particularly, the invention relates to a method for investigating the gene variability and the functional variability of HBV.

Despite the existence of a vaccine against the hepatitis B virus, chronic infection with this virus remains a worldwide public health problem. It is estimated that, currently, 2 billion individuals have been infected with HBV and that 450 million are chronic carriers of this virus throughout the world. Chronic infection with HBV is associated with serious complications such as liver cirrhosis and hepatocellular carcinoma. Hepatocellular carcinoma is one of the eight most common cancers in the world.

Several approved treatments against chronic HBV infection exist : alpha-interferon, which is an immunostimulator that makes it possible to treat approximately 40% of chronic carriers, and nucleoside analogues, which are viral replication inhibitors. Currently, two analogues are used : Lamivudine and Adefovir. These nucleoside analogues are inhibitors specific for the viral polymerase of HBV, but also for that of other viruses, such as the human immunodeficiency virus (HIV) against which they were initially developed. However, as in the case of treatment against HIV, these compounds have rapidly been confronted with the emergence of resistant viral strains of HBV, from 50% to 65% of resistance after 2 to 3 years of treatment for Lamivudine, and from 2 to 3% of resistance to Adefovir after 2 years of treatment. Although the rate of resistance to the latter product is low, it unfortunately appears that the virological response with this medicinal product is variable and that some patients appear to respond weakly or not at all to this drug.

There are more than ten or so new antiviral agents in clinical trials or at the preclinical development stage against HBV. However, as for the two molecules placed on the market, resistances to treatments are already appearing.

As for HIV, it will therefore rapidly be necessary to evaluate the resistance of the viral strains that evade the treatment in patients. This resistance may be correlated with the known mutations in the polymerase and analysed by genotyping techniques and/or evaluated directly by phenotyping in cell expression systems.

Various methods for the genotypic analysis of HBV viral strains resistant to the current antiviral agents were known in the art.

The most laborious and the oldest method is direct sequencing of the regions of the virus exhibiting the mutations described in the literature. This sequencing only makes it possible to analyse the major population present in a patient. For these strategies, various subgenomic PCR techniques exist for amplifying the polymerase regions involved in resistance, such as the C domain, where the main mutations responsible for resistance to Lamivudine (L180M and M2041 or M204V) and the new mutation A181V associated with resistance to Adefovir are located, or the D domain of the polymerase, where the N236T mutation for resistance to Adefovir is located (Angus et al., 2003; Villeneuve et al., 2003).

More recently, differential hybridization techniques on specific probes have been developed, such as the Line Probe assay (INNO-LiPA, Innogenetics), which makes it possible, after subgenomic fragment amplification, to determine the genetic profile (Lok et al., 2002). Based on a similar principle of differential hybridization, chips are in the process of being developed, which will make it possible, on the same chip, to search for predefined mutations (BioMérieux/Affimétrix).

However, for the drugs currently in clinical development, new emerging mutations associated with resistances have already been described, such as for Tenofovir (Sheldon; 44th ICAAC Meeting: Washington, DC, Oct 30-Nov 2, 2004). In certain cases, the mutations already described can lead to cross resistances, for instance between Lamivudine and nucleoside analogues such as Telbivudine, Entecavir (Tenney et al., 2004) or Emtricitabine for mutations at L180M and/or M204V (Delaney, (ICAR, 2004), (AASLD, 2004)). The appearance of these new mutations and the cross resistances observed mean that genotyping assays must constantly adapt and, ultimately, it will be necessary to develop, as for HIV, algorithms capable of divining a virtual genotype for resistance as a function of the mutations observed. These algorithms must also evolve with the appearance of new drugs and potentially of new resistance mutations.

However, since all these techniques are based on the amplification of subgenomic HBV DNA fragments originating from patients, they are not compatible with phenotyping assays and tests for analysing the replicative capacity of these viruses, which require amplification of the whole of the HBV genome.

The development of a phenotypic and replicative capacity test requires that a certain number of technical problems be solved.

The first difficulty is that of producing HBV *in vitro.* This virus replicates via a reverse transcription step that requires the synthesis of a pregenomic RNA (pgARN) of length corresponding to 1.1 units of viral genome. This step is only possible on transcription units at least equal to 1.1 genome in size (for example dimers) or on circular DNAs, the natural state of HBV in the cell nucleus in the form of supercoiled DNA (cccDNA).

Unlike other types of viral infection (for example, HIV), there are no HBV-infectable cell line models compatible with use in phenotypic tests (Gripon et al., 2002).

Various strategies for producing HBV have been developed in a cell system. Plasmid constructs comprising dimeric forms of the virus have been constructed and transfected (Ladner et al., 1997; Sells et al., 1987). Other strategies have made it possible to construct an HBV genome under the control of a strong promoter stably or transiently expressed in human hepatoma lines. These constructs have made it possible, by site-directed mutagenesis or by PCR cassette exchange, to study the influence of certain mutations on viral replication and their impact on resistance to Lamivudine, for example (Allen et al., 1998; Bock et al., 2002; Pichoud et al., 1999; Seigneres et al., 2002). However, all these strategies are based on laboratory constructs which do not make it possible to transfer and to study the overall viral population found in a patient in whom treatment is failing.

In order to make it possible to evaluate the phenotypic resistance of HBV using a patient's serum, three main strategies have been developed:

### Strategy 1: Whole genome PCR amplification and detection by Southern blotting

Gunther et al. (Gunther et al., 1995) describe a PCR capable of amplifying the entire HBV genome. This PCR product, once transfected *in vitro* into hepatoma cell lines, circularizes *in cellulo* and becomes the transcription matrix for the virus. In fact, this PCR system makes it possible to introduce the Sap I restriction enzyme site into each primer, which site, once digested and religated, results in a whole circular HBV DNA, the natural state of the virus in the cell.

This technique for amplifying the viral population from a serum makes it possible to obtain an exact replica of the entire viral quasi-species found in a patient.

However, this strategy results in very low replication levels *in vitro.* In fact, the Sap I enzyme used is relatively ineffective during cleavage and appears to only partially allow recircularization *in cellulo.* This strategy can therefore only be carried out with difficulty, with visualization by Southern blotting (as described by Günther et al.), and in fact only makes it possible to have an estimation of the replicative capacity of the viruses. The replication levels obtained are not sufficient to make it possible to perform a phenotyping assay for evaluating resistance to antiviral agents.

### Strategy 2: Cloning of patient genomes under a strong promoter, detection by Southern blotting.

The new cloning strategies (Durantel et al., 2004 ; Yang et al., 2004; WO 2004/029301) make it possible to amplify the genomes originating from patients by simple or multiple PCRs, and to strongly express the pgRNA of 1.1 genome units necessary for replication, by using strong eukaryotic promoters in human hepatoma cells. The HBV transcription and replication levels are then much higher than the levels observed with the dimer constructs or the Günther strategy in which the HBV is synthesized under the control of its natural promoter (Durantel et al., 2004 ; WO 2004/029301). However, these high transcription levels may not reflect the real replicative capacity of the viral population in the patients.

In addition, these cloning techniques are more complex and less rapid than the whole genome PCR technique and systematically result in the creation of chimeric genomes made up of fragments that do not necessarily come from the same starting genomes in the patients. For certain genotypes (B and some C genotypes, in the majority in Asia), the cloning technique described by Durantel et al. is complicated by the presence of an additional restriction site, in these genotypes for the Nco I enzyme used for the cloning. Finally, certain genotypes (G genotype) are found to show weak replication *in vitro,* even with this technique, and can be analysed by Southern blotting only with great difficulty, especially for the mutant viruses having a low replicative capacity.

In summary, these strategies make it possible to laboriously obtain a mixture of vectors strongly expressing mosaic genomes of HBV originating from the patient. The influence of the existing mutations on a part of the genome other than the polymerase gene is not taken into consideration and this strategy makes it difficult to evaluate the real replicative capacity of the viral population.

Another limitation of these phenotyping assays (Strategies 1 and 2) is the time taken to carry them out. The techniques for measuring viral replication are carried by Southern blotting (DNA extraction, migration on agarose gel and transfer onto nylon membranes), and then by labelling of the DNA with phosphorus 32 and quantification by means of a phosphoimager (Durantel et al., 2004). This technique is very laborious, relatively insensitive, and long. It requires large amounts of cell cultures and, as a result, it is not transposable to a service activity such as large-scale phenotyping. In addition, the use of radioactive material requires protective equipment and potentially dangerous handling.

The visualization by Southern blotting is therefore very long and results in tests that are carried out in 3 to 4 weeks. This amount of time does not make them compatible with a therapeutic decision which must be adapted for patients, in whom therapy is failing, with potentially evolving hepatic pathologies.

### Strategy 3: Whole genome PCR amplification and detection by real time PCR

Some authors have more recently used a method for quantifying HBV produced in culture, by real time PCR, a method more sensitive than Southern blotting (Lada et al., 2004). This team has thus been able to carry out a phenotyping assay for viruses originating from patients after amplification by the Günther method. This analysis remains limited due to the culture format used, which does not make it possible to analyse a large number of samples. In addition, in order to avoid having interference between the HBV DNA transfected and the DNA produced, the real time PCR analysis is carried out on the culture supernatant after treatment with the various drugs. However, an analysis in culture supernatant is not optimal since certain HBV viral populations can be defective and not produce complete and mature virions in cell culture. Furthermore, this analysis can be contaminated by the residual DNA transfected at the start or released by the cells in the event of drug toxicity. Finally, this team carried out a cloning step after the amplification by the Günther method and selected only one clone with strong replication *in vitro;* the analysis performed here does not therefore reflect the real sensitivity of the quasi-species found in the patient.

In addition to these three phenotyping strategies, stable lines of HBV under a strong promoter, cultured in the 96-well culture format, with detection of the HBV production by real time PCR, have been described (Oral presentation: WE Delaney, Gilead Sciences, Foster City, CA USA at the 17th ICAR, May 2 - May 6, 2004, Tuscon, AZ ; ICAR, 2004).

For this study, the human hepatoma cell lines were cultured in a 96-well plate format. This format is more compatible with high throughput studies that include numerous samples. On the other hand, the authors use stable lines expressing various known HBV mutants, which lines are constructed in the laboratory with viruses that do not originate from patients. This analysis in fact only represents an *in vitro* analysis of cross resistances between Lamivudine and the other drugs under development. These laboratory constructs also make it possible to carry out rapid screening of new drugs on known mutants.

The use of this type of culture format for quantifying, by real time PCR, viral production after transfection of HBV originating from a patient poses a problem of specificity. In fact, in the PCR techniques or the techniques for cloning under a strong promoter (strategies 1 to 3) described above, the genomes and/or constructs originating from patients are directly transfected into the human hepatoma cells in large amounts. This large amount of DNA becomes a problem for the quantification by real time PCR of the virions produced. These strategies, in order to be usable in a format compatible with large scale use, therefore require the development of methods making it possible to eliminate the transfective residual DNA in order to analyse only the virions produced.

The aim of the present invention is to provide a novel, easy to use and more rapid strategy for obtaining, from a biological sample from a patient infected with HBV, a measure of the replicative capacity and of the genotypic and phenotypic resistance of the virus, so as to have a better understanding of the patient's situation and therefore to more effectively direct the therapy.

The method according to the invention permits to study the global viral population present in patients and makes it possible to analyze the impact of any mutations (known or unknown) present in the quasi-species by assaying susceptibility differences to any drugs (currently approved or in different phases of development). Eventual combination of drugs and search for synergic or additive effects may further be studied.

The invention provides a method that is based on the production of an amplicon which makes it possible to evaluate HBV strains resistant to antiviral agents by analysing, using the same amplicon obtained from a patient, the genotype (presence of known mutations), the phenotype and the replicative capacity. This novel method is sensitive and rapid and makes it possible to directly study the viral quasi-species originating from a patient under the control of their own promoter. The use of an amplicon containing the natural promoter of HBV guarantees to obtain the closest *in vitro* replication capability compared to the *in vivo* replication. Moreover, the method avoids creating any chimeric virus which ensures that the influences of all the mutations present on a viral genome are taken into account when evaluating the drug sensitivity and the replicative capacity. At last, the method according to the invention is more convenient to use in current laboratories and can be finally scaled up by automatic machines to process a lot of samples.

### Definitions

The term "hepatitis B virus" or "HBV" denotes the type species of the genus Orthohepadnavirus, family Hepadnaviridae. HBV nucleic acid is a partially double stranded and single stranded circular DNA. The total genome length is about 3020-3320 nucleotides (nt) (for the full length strand), or 1700-2800 nt (for the short length strand). Hepatitis B virus (HBV) has been classified into eight genotypes (A-H) based on genome sequence divergence. As used herein, HBV is meant for any subtype, strain, or variant, e.g. serotype *ad, adr, adw, adyw, ar, ayw.* In the context of the invention, nucleotide positions are indicated by reference to the HBV genome sequence, serotype *adrq,* deposited at GenBank under accession number X75665 (SEQ ID NO.1). The nucleotides positions in the sequence SEQ ID NO.1 correspond to the same nomenclature as defined by Norder et al., 1994.

As used, herein, the term "quasi-specie" denotes the total population of HBV virus found in a patient. The quasi-specie is due to minor genetic differences found in this population in an individual infected with a single genotype. This quasi-specie changes in an individual over time, for example under drugs treatment, whereas genotypes do not.

In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989") ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et al. (eds.), Current Protocols in Molecular Biology, John Wiley & Sons, Inc. (1994).

A "nucleic acid molecule" refers to any nucleic acid : it may be synthetic or not, recombinant or naturally occurring, linear or circular. It may be either in single stranded or in double stranded form. These nucleic acid molecules include genomic DNA, cDNA or RNA. Unless otherwise specified, sequences are described according to the normal convention of giving only the sequence in the 5' to 3' direction along the non-transcribed strand of DNA (i.e., the strand having a sequence homologous to the mRNA).

A "promoter" or "promoter sequence" is a DNA regulatory region capable of binding RNA polymerase in a cell and initiating transcription of a downstream (3' direction) coding sequence.

The terms "vector", "cloning vector" mean the vehicle in which a DNA sequence can be introduced. Preferably, the vector according to the invention is a prokaryotic or eukarotic vector that makes it possible to amplify the cloned sequence (e.g. a HBV genomic amplicon) and/or select HBV sub-populations. A common way to introduce a DNA sequence into a vector involves the use of restriction enzymes that cleave DNA at specific sites (specific groups of nucleotides) called restriction sites. Similarly, the sequence introduced in the vector may be extracted from the vector using a restriction enzyme. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids, pTriex plasmids (Novagen, Inc., Madison, Wl), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, e.g. antibiotic resistance, and one or more expression cassettes.

As used herein, the term "oligonucleotide" refers to a nucleic acid sequence, which can be used as primer in an amplification procedure or as probe in a method of detection. In the context of the invention, these oligonucleotides consists of at least 10, preferably at least 15, preferably at least 20, and more preferably at least 30 nucleotides, preferably no more than 100 nucleotides, more preferably no more than 50, also preferably no more than 40 nucleotides that are hybridizable to a genomic DNA molecule, a cDNA molecule, or a mRNA molecule encoding, or other nucleic acid of interest.

As used herein, the term "homologous" in all its grammatical forms and spelling variations refers to the relationship between proteins that possess a "common evolutionary origin". Such proteins (and their encoding genes) have sequence homology, as reflected by their sequence similarity, whether in terms of percent similarity or the presence of specific residues or motifs at conserved positions. Accordingly, the term "sequence similarity" in all its grammatical forms refers to the degree of identity or correspondence between nucleic acid or amino acid sequences that may or may not share a common evolutionary origin. In a specific embodiment, two DNA sequences are "substantially homologous" or "substantially similar" when at least about 80 %, and most preferably at least about 90, or 95 %, of the nucleotides match over the defined length of the DNA sequences, as determined by sequence comparison algorithms, such as BLAST, FASTA, DNA Strider, etc. Sequences that are substantially homologous can be identified by comparing the sequences using standard software available in sequence data banks, or in a Southern hybridization experiment under, for example, stringent conditions as defined for that particular system.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., supra). A specific example of hybridizable nucleic acid molecule is an oligonucleotide complementary to a nucleic acid molecule, or a fragment of a nucleic acid molecule. As used herein, a first sequence is "complementary" to a second sequence where the totality of the first sequence perfectly matches the second sequence.

The conditions of temperature and ionic strength determine the "stringency" of the hybridization. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm : melting temperature) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridization with shorter nucleic acids, i.e., oligonucleotides, the position of mismatches becomes more important, and the length of the oligonucleotide determines its specificity (see Sambrook et al., supra, II.7-11.8). A minimum length for a hybridizable nucleic acid is at least about 10 nucleotides, preferably at least about 15 nucleotides, and more preferably the length is at least about 20 nucleotides.

Typically, low stringency hybridization conditions, correspond to a Tm of 55°C, and, e.g., 5x SSC, 0.1 % SDS, 0.25 % milk, and no formamide ; or 30 % formamide, 5x SSC, 0.5 % SDS. Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. For instance, "high stringency" may refer to hybridization and/or washing conditions at 68°C in 0.2 X SSC, at 42°C in 50 % formamide, 4 X SSC.

"Amplification" of DNA or "amplifying DNA" as used herein denotes the increase of the concentration of a particular DNA sequence within a mixture of DNA sequences. The amplification step may be carried out by any method using conventional methods of enzymatic amplification of DNA, such as in particular the Transcription Mediated Amplification (TMA) described in US 5,399,491, the Rolling Circle Amplification (RCA) described for instance by Lizardi et al. (Lizardi et al., 1998), or by Zhang et al. (Zhang et al., 1998) or the polymerase chain reaction (PCR) technique as described by Saiki et al. (Saiki et al., 1988) and in European patents EP 0 200 362 and EP 0 201 184, or alternatively the techniques derived from the latter and any other method desired for amplifying nucleic sequences *in vitro.* Preferably, the method of amplification according to the invention is PCR.

As used herein, the term "primer" refers to the function of the oligonucleotide. A primer is an oligonucleotide used for amplifying a target sequence typically by extension of the oligonucleotide after hybridization to the target sequence.

The term "amplicon" is meant the product of PCR; or more generally a piece of DNA that has been synthesized using amplification techniques. A "HBV genomic amplicon" denotes a DNA sequence comprising the whole genome of HBV and generated by an amplification technique.

A "restriction enzyme" denotes an endonuclease that binds at a recognition site and then cleaves a DNA strand at a fixed position relative to the recognition sequence (type II restriction enzyme). The "recognition sequence" is the specific nucleotide sequence to which a restriction enzyme binds prior to cutting the DNA backbone. Recognition sequences are generally 4 to 8 base pairs in length, and are often palindromic - that is, they read the same backwards and forwards when they are read in the 5' - 3' direction -, and the recognition sequence is the same on both strands of the DNA. The "cutting site" is the specific nucleotide sequence at which the restriction enzyme cuts. In some cases, the cleavage points occur exactly on the axis of symmetry of the palindromic restriction site, giving products which are blunt-ended. Some restriction nucleases produce staggered cuts, which leave short single-stranded tails at the two ends of each fragment, known as "cohesive ends". The cohesive ends generated by restriction enzymes allow any linear DNA to circularize.

Positions of cleavage relative to the recognition sequence depend on the enzyme. For instance for the Sap I enzyme (type IIS restriction enzyme), the cutting site is outside from the recognition sequence:

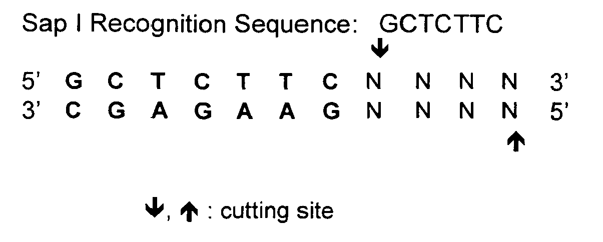

Preferably, the restriction enzyme according to the invention has its cutting site contained within its recognition sequence. Accordingly, as used herein a "restriction site" preferably denotes a nucleotide sequence which consists of the recognition sequence for a restriction enzyme and which contains the cutting site of said enzyme.

As used herein, the term "genotyping" denotes determining the genomic or a subgenomic sequence of a HBV strain, such as in particular determining the presence of specific known mutations.

"Phenotyping" is intended for the determination of the resistance of a HBV strain to one or several antiviral drugs, in particular drugs interacting with HBV replication.

A "patient" denotes a mammal, preferably a human, likely to or known to be infected with HBV. The patient may be undergoing treatment of HBV infection, or not receiving treatment or experiencing failure of treatment.

A "biological sample" according to the invention may be a blood, serum, plasma, or any other biological fluid. The sample may also be a biopsy of an organ that may contain HBV. It may also come from a viral cell culture or from an animal that may be carrying HBV.

### Method of production of HBV genomic amplicons, primers and kits

The aim of the present invention is to provide a novel strategy for obtaining, from a biological sample originating from a patient, a measure of the genotypic and phenotypic resistance (i.e. resistance to antiviral drugs) and of the replicative capacity of the HBV viruses in order to have a better understanding of the patient's situation and to more effectively direct the therapy.

This aim is achieved by generating genomic amplicons which are usable for genotypic, phenotypic resistance and replicative capacity analysis. These amplicons are generated by amplification of HBV quasi-species genomic nucleic acids obtained from a patient.

Thus the invention provides a method for preparing HBV genomic amplicons from a biological sample of a patient, which are usable for genotypic, phenotypic and replicative capacity analysis, said method comprising the steps consisting of :
a) extracting HBV nucleic acid from the biological sample;
b) amplifying the HBV nucleic acid with a pair of primers :
   - that are selected to obtain amplicons comprising the entire HBV genome, and
   - that comprise a copy of a restriction site that is not present or is present only once in a consensus sequence of HBV genome of all HBV genotypes,
   wherein said restriction site is cut by a restriction enzyme within the recognition sequence for said restriction enzyme ;
c) digesting the amplicons with the restriction enzyme which cuts said restriction site;
   and
d) purifying the digested amplicons.

The HBV nucleic acid may be DNA or RNA. The extraction step a) may be carried out by any method known in the art. These methods comprise conventional methods by precipitation and methods using silica columns or chromatography columns. Use of the Ultrasens viral nucleic acid extraction kit from Qiagen is ideal for this step.

Where HBV nucleic acid is RNA, a reverse transcription step may be carried out prior to amplification. Reverse transcription of RNA sequences is readily known by the one skilled in the art.

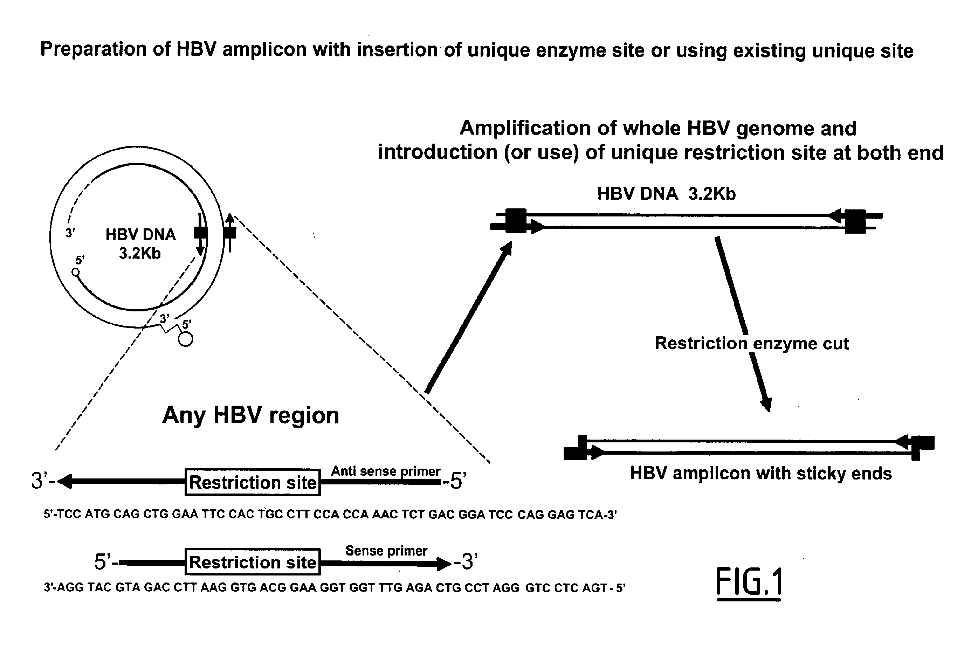

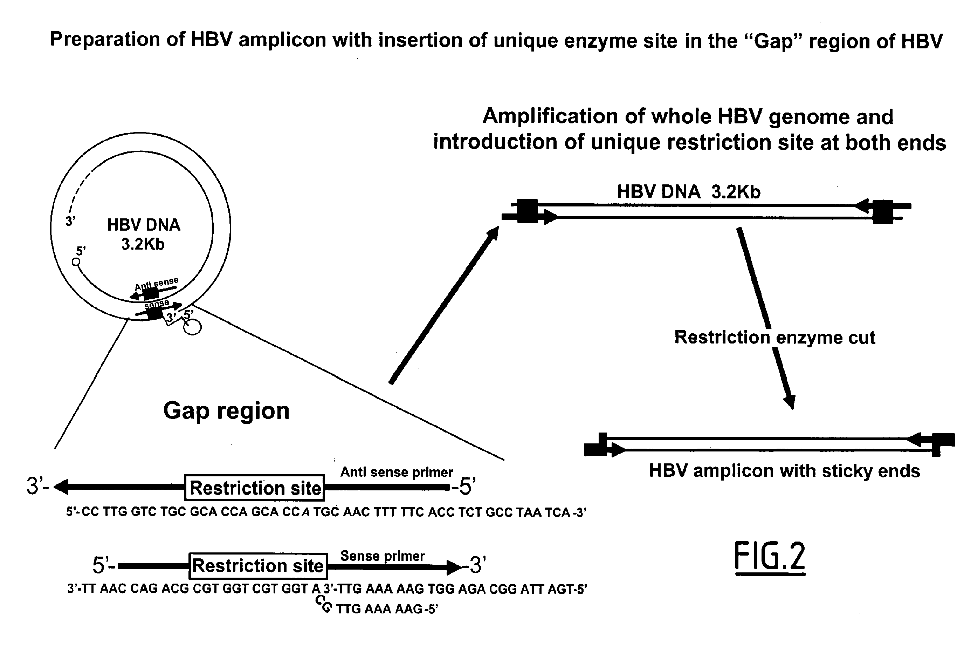

The invention is based on the selection of pairs of PCR primers that make it possible both to amplify whole HBV genomes and to produce amplicons that comprise two copies of a restriction site specific for an enzyme that allows recircularization of the genomes in a cell, and results in high replication levels. The amplicons generated by amplification are double stranded linear DNAs that comprise two copies of said restriction site, one copy being localized at both ends of the amplicons. A diagrammatic representation of the principle of the method of production of HBV genomic amplicons according to the invention is shown on Figure 1 and Figure 2.

On one hand, said restriction site may be naturally present only once in a consensus sequence of HBV genome of all genotypes; accordingly the primers are selected to hybridize with a region of HBV genome that contains said restriction site. On the other hand, the primers may be designed to introduce in the amplicons a restriction site which is not present in a consens us sequence of HBV genome of all genotypes.

The primers according to the invention preferably have the following characteristics :
(i) they comprise a copy of a restriction site present only once in a consensus sequence of HBV genome of all genotypes, or they comprise a copy of restriction site which is not naturally present in HBV consensus sequences of all HBV genotypes and which is then created in the amplified HBV genome without impairing the genetic content thereof (it will be obvious for the one skilled in the art that depending on whether the primer is a forward primer or a reverse primer, the primer may comprise a sequence which is the sense sequence of the restriction site or the antisense sequence of said restriction site);
(ii) they contain a restriction site which allows recircularization of the DNA amplified in the cell ; i.e. digestion of the linear amplicons generated by PCR amplification, with the restriction enzyme specific for the single restriction site, should generate amplicons with 5' and 3' ends allowing recircularization. That means 5' and 3' ends with cohesive ends corresponding to the cutting site of the restriction enzyme used. This is possible only with enzymes which cut in their one recognition site.

Description of restriction enzymes and their recognition and cutting sites are publicly available. Reference may be made for instance to Sambrook et al. (1989), or to databases such as Rebase® (http://rebase.neb.com/rebase/rebase.html), or the Restriction Enzymes Web Page (http://internahmed.wustl.edu/divisions.enzymes.INDEXX.HTM).

Preferably each primer contains a single copy of said restriction site. Should the primers contain more than one copy of the restriction site, these copies would be removed from the amplicons upon digestion with the appropriate restriction enzyme.

As used herein, the term "HBV consensus sequence" or "consensus sequence of HBV genome" is meant a single sequence or sequences of nucleotides which is (are) designed to have a high degree of homology with a set of known HBV genomic sequences, preferably sequences of all known HBV genotypes. A HBV consensus sequence may be readily determined by the one skilled in the art by sequence comparison of known HBV genomic sequences, e.g. with sequence comparison algorithms such as BioEdit Software (Hall, 1999) that are publicly available. For instance, a HBV consensus sequence may be a genomic sequence of HBV which has been computer-designed so that for each nucleotide position, the nucleotide is present in at least 50%, preferably 60% of all HBV genomes for each genotype. According to an embodiment, the consensus sequence corresponds to a consensus sequence of the majority of genomes found in one genotype of HBV. An example of HBV consensus sequences is shown in SEQ ID No.2 to 9 for genotypes A to H, respectively. Accordingly, a HBV consensus sequence of all genotypes denotes a set of consensus sequences wherein each sequence is a consensus sequence of a single HBV genotype (genotypes A to H thus 8 consensus sequences). According to another embodiment, a single consensus sequence corresponds to a consensus sequence of the majority of genomes found in all genotypes of HBV.

Where said restriction site contained in the primers (and in the generated amplicons) is not present in consensus sequences of HBV genome of all genotypes , said restriction site preferably meets one or more of the following criteria:
(i) it is substantially absent of all HBV sequences ;
(ii) it does not introduce more than 3 nucleotide changes in the HBV genomic sequence.

The introduction of a restriction site which is substantially absent of all known HBV sequences is aimed at enabling to amplify and purify a maximum number of genomic nucleic acids of HBV quasi-specie isolated from the biological sample of the patient. If the introduced restriction site is already present in the genome of a HBV quasi-specie, the amplicons generated for this quasi-specie will comprise three copies of the restriction site (two copies introduced at both ends of the amplicons by the primers, and one inner restriction site). Accordingly, the digestion of such an amplicon with the corresponding restriction enzyme will produce two digestion products, none of them having the length of a whole HBV genome. Accordingly it would not be possible to achieve preparation of a complete HBV genomic amplicon for the quasi-specie. Preferably, a "restriction site which is substantially absent in all HBV sequences" denotes a restriction site which can not be found more than 40 times, preferably not more than 30 times, still preferably more not more than 20 times, or also preferably not more than 10 times, in all HBV sequences published, or in a set of known HBV sequences.

Similarly, the selection of primers containing a copy of a restriction site which is present only once in consensus sequences of HBV genome of all genotypes makes it possible to amplify and purify genomic nucleic acids of a maximum number of HBV quasi-species isolated from the biological sample of the patient.

Preferably, the primers are hybridizable to the HBV "gap" region, i.e. the region defined by nucleotide positions 1800 to 1850 of SEQ ID No.1. In particular, the primers comprise a nucleotide sequence complementary to the HBV gap region, e.g. the primers comprise the nucleotide sequence complementary to the whole HBV gap region or to a fragment of HBV gap region which comprises at least 12, preferably at least 15, still preferably at least 20 nucleotides, and preferably no more than 50 nucleotides, still preferably no more than 40 nucleotides.

The HBV gap region contains restriction sites, but none of them is a site which allows recircularization and which is present only once. Accordingly, where the primers used for amplification are hybridizable to the HBV "gap" region, they are designed to introduce in the amplicon a restriction site (preferably a single restriction site) which is not present in consensus sequences of HBV genomes of all HBV genotypes.

Preferably, the restriction site introduced by means of the primers is a site for an enzyme selected from the group consisting of BssH II, Cla I, Mlu I, Nhe I, Not I, Pvul, Rca I, Sun I (BsiW I), and Nar I. Still preferably, the site for an enzyme selected from the group consisting of BssH II, Cla I, Mlu I, Nhe I, Not I, Pvul, Rca I, Sun I (BsiW I), and Nar I is introduced in HBV gap region.

According to a preferred embodiment, the pair of primers introduces a restriction site for the Nar I enzyme. Preferably, the pair of primers introduces a restriction site for Nar I into the "gap" region of HBV genome, i.e. the region defined by nucleotides 1800 to 1850 of the sequence of HBV shown in SEQ ID No.1. Thus, a preferred pair of primers according to the invention consists of primers comprising the nucleotide sequence of the Nar I restriction site (GGCGCC, SEQ ID No.41) and a nucleotide sequence complementary to the HBV gap region.

The pairs of primers may have the sequences selected from the group consisting of SEQ ID No.42 and SEQ ID No.43, SEQ ID No.44 and SEQ ID No.45, SEQ ID No.11 and SEQ ID No.12, SEQ ID No.14 and SEQ ID No.15, SEQ ID No.18 and SEQ ID No.19, SEQ ID No.22 and SEQ ID No.23, SEQ ID No.26 and SEQ ID No.27, SEQ ID No.30 and SEQ ID No.31, SEQ ID No.34 and SEQ ID No.35, and SEQ ID No.38 and SEQ ID No.39 as defined below.

The nucleotides making up the restriction site are underlined. The modified bases as compared to the sequence published by Norder et al. (1994) or the sequence deposited in Genbank X75665 (SEQ ID No.1), are shown in bold. The "A" of the ATG codon (in bold italic) of the pregenomic RNA included in all sense primers is located at a position corresponding to position 1814 of the sequence SEQ ID No.1. The corresponding antisense "T" is also shown in bold italic in the reverse primers. N represent any nucleotide A, C, G or T, and x is a integer which is at least equal to 3, preferably comprised between 3 and 20, still preferably comprised between 4 and 10.

The (N)x stretch in the oligonucleotide sequences below are eliminated from the amplicons upon cleavage of the restriction site with the corresponding enzyme. The sequence of the (N)x stretch is advantageously defined so as to optimize amplification.

For BssH2 : site : GCGCGC (SEQ ID No.10)
sense 5'-(N)xGC GCG CCA GCA CCA TGC AAC TTT TTC ACC TCT GCC TAA
TCA-3' (SEQ ID No.11), for instance N = C and x = 4; and
antisense 5'-GTG AAA AAG TTG CAT GGT GCT GGC GCG C(N)x-3' (SEQ ID
No.12), preferably (N)x is AGA.

For Cla I : site ATCGAT (SEQ ID No.13)
sense 5'-(N)xAT CGA TGC AAC TTT TTC ACC TCT GCC TAA TCA-3' (SEQ ID
No.14), for instance N = C and x = 4; and
antisense 5'-GTG AAA AAG TTG CAT CGA T(N)x -3' (SEQ ID No.15), preferably
(N)x is CTGGTGCG (SEQ ID No.16).

For Mlu I site : ACGCGT (SEQ ID No.17)
sense 5'-(N)xAC GCG TAC CA TGC AAC TTT TTC ACC TCT GCC TAA TCA-3'
(SEQ ID No.18), for instance N = C and x = 4; and
antisense 5'-GTG AAA AAG TTG CAT GGT ACG CGT (N)x -3' (SEQ ID No.19),
preferably (N)x is CGCAGA (SEQ ID No.20).

For Pvu I : site CGATCG (SEQ ID No.21)

sense 5'-(N)x CGA TCG CCA TGC AAC TTT TTC ACC TCT GCC TAA TCA-3' (SEQ
ID No.22), for instance N = C and x = 3; and
antisense 5'-GTG AAA AAG TTG CAT GGC GAT CG (N)x-3' (SEQ ID No.23),
preferably (N)x is GCGCAGA (SEQ ID No.24).

For Rca I : site TCATGA (SEQ ID No.25)
sense 5'-(N)x TCA TGA CCA TGC AAC TTT TTC ACC TCT GCC TAA TCA-3' (SEQ
ID No.26), for instance N = C and x = 3; and
antisense 5'-GTG AAA AAG TTG CAT GGT CAT GA(N)x-3' (SEQ ID No.27),
preferably (N)x is TGCGCAGA (SEQ ID No. 28).

For Nhe I : site : GCTAGC (SEQ ID No.29)
sense 5'-(N)xGC TAG CAC CAT GCA ACT TTT TCA CCT CTG CCT AAT CAT-3'
(SEQ ID No.30), for instance N = C and x = 4; and
antisense 5'-GTG AAA AAG TTG CAT GGT GCT AGC (N)x-3' (SEQ ID No.31),
preferably (N)x is GCGC (SEQ ID No.32).

For Not I : site : GCGGCCGC (SEQ ID No.33)
sense 5'-(N)xGC GGC CGC ACC ATG CAA CTT TTT CAC CTC TGC CTA ATC-3'
(SEQ ID No.34), for instance N = C and x = 4; and
antisense 5'-GTG AAA AAG TTG CAT GGT GCG GCC GC(N)x -3' (SEQ ID No.35),
preferably (N)x is GCAG (SEQ ID No. 36).

For Sun I (BsiW I) : site : CGTACG (SEQ ID No.37)
sense 5'-(N)xCG TAC GAG CAC CAT GCA ACT TTT TCA CCT CTG CCT AAT
CAT-3' (SEQ ID No.38), for instance N= C and x =4; and
antisense 5'-GTG AAA AAG TTG CAT GGT GCT CGT ACG (N)x-3' (SEQ ID No.39),
preferably (N)x is CAGA (SEQ ID No.40).

For Nar I site : GGCGCC (SEQ ID No.41)
sense 5'- (N)xG GCGC CAT GCA ACT TTT TCA CCT CTG CCT AAT CAT-3' (SEQ
ID No.42), and
antisense 5'- GTG AAA AAG TTG CAT GGC GCC (N)x-3' (SEQ ID No.43).

Preferably the sequences used for Nar I site are :

sense 5'- GGG G GCGC CAT GCA ACT TTT TCA CCT CTG CCT AAT CAT-3'
(SEQ ID No.44)

antisense 5'- GTG AAA AAG TTG CAT GGC GCC GGT GCG-3' (SEQ ID No.45).

These primers may be used in any suitable amplification method, preferably PCR. The choice of the appropriate conditions (e.g. temperature, buffers) are within the ordinary skills of the one skilled in the art. An example of PCR amplification is described in example 1 below.

According to a preferred embodiment the primers used for amplification have the sequences SEQ ID No.42 and SEQ ID No.43 or SEQ ID No.44 and SEQ ID No.45.

Optionally, the amplicons obtained after amplification, e.g. PCR amplification, are purified by conventional amplified product purification techniques, of the silica column type, prior to digestion.

The digestion step (c) is performed using the appropriate restriction enzyme, i.e. the one that cuts the restriction sites contained at both ends of the amplicons. The digestion may be readily carried out by the one skilled in the art, for instance by following the restriction enzyme manufacturer's instructions.

Once digested, the amplicons are purified by conventional purification techniques. For instance, the purification step (d) may be carried out using a silica column, type like Qiagen, or using standard phenol Chloroform procedures, for instance Qiaquick PCR purification Kit from Qiagen.

The method for preparing HBV genomic amplicons according to the invention makes it possible to obtain the most representative population of the virions present in a patient, without creating any chimeric virus, and where said virions replicate under their natural promoter. These points make it possible to guarantee that the analysis of the replicative capacity of these viruses will be optimal and that all the mutations present on each genome will be taken into consideration by a phenotyping assay. Furthermore, the fact that the entire viral genome is amplified makes it possible to readily carry out genotyping assays for the regions involved in viral resistance.

Additionally, the purified amplicons obtained in step d) may be cloned into any vector allowing their incorporation. Thus, the method of preparing HBV genomic amplicons according to the invention may optionally comprise a further step (e) consisting of cloning the amplicons obtained in step (d) in a prokaryotic or eukaryotic vector, and amplifying the cloned amplicons and/or selecting amplicons of viral sub-populations, and extracting and purifying the amplicons amplified and/or selected from the vector.

This cloning step firstly makes it possible to carry out an amplification of the genetic material obtained in step (d) in order to prepare storage banks of this material and/or to use this material for the subsequent steps (i.e. genotyping, analysis of replicative capacity and phenotyping). In this case, the cloned amplicons may be digested with the enzyme that cuts the restriction sites which are present in the amplicons at their extremities and extracted from the cloning vector by conventional techniques. Secondly, the cloning step makes it possible to individualize the viral genomes present in a biological sample of a patient and thus to be able to carry out a study of the genotypic and phenotypic resistances of each genome present. This step also makes it possible to carry out an evaluation of the quasi-species present in a patient by defining the relative proportions of the genotypes and phenotypes present. In particular, viral sub-populations may be isolated among the HBV quasi-specie e.g. to study the becoming of said sub-populations in the course of antiviral treatment (i.e. sensitivity to the treatment or emergence of resistance).

The invention also relates to a pair of primers which may be used to implement the method of preparing HBV genomic amplicons according to the invention.

The invention further provides a kit for preparing HBV genomic amplicons from a biological sample of a patient, which kit comprises:
- at least a pair of primers, as defined above;
- means for amplifying a HBV nucleic acid.

According to a preferred embodiment, said a pair of primers comprise the sequence of (or complementary to) a restriction enzyme site present which is not naturally present in HBV consensus sequence, in particular a site for an enzyme selected from the group consisting of BssH II, Cla I, Mlu I, Nhe I, Not I, Pvul, Rca I, Sun I (BsiW I), and Nar I.

Preferably, the means for amplifying HBV nucleic acid are means for amplification by Polymerase Chain Reaction.

### Method of genotyping

The HBV genomic amplicons prepared according to the method of amplification described above may advantageously be used to assess the genetic variability of the HBV strains present in a biological sample.

The amplicons obtained in step (d) or (e) of the above method of preparing of HBV genomic amplicons can be either directly or indirectly used for the genotypic analysis of known mutations of HBV. For instance, these analyses can be carried out on the amplicons obtained in step (d) or (e) directly, or after subgenomic PCR steps, if necessary.

Thus the invention relates to a method of genotyping of HBV strains present in a biological sample of a patient, which comprises the step consisting of genotyping the amplicons obtained by a method of preparing HBV genomic amplicons as described above.

The genotyping may be carried out by direct sequencing or by other techniques readily known to the one skilled in the art, such as differential hybridization techniques (Lok et al., 2002), in order to search for the presence of mutations that may or may not have been described as being associated with viral resistance to antiviral products. Where direct sequencing is used for genotyping, it may be carried out on the amplicons directly obtained in step (d) of the above method of preparation of HBV genomic amplicons. Where differential hybridization is used for genotyping, it is advantageously performed on subgenomic fragments of HBV DNA.

### Method of determining replicative capacity and/or phenotyping resistance

The HBV genomic amplicons prepared with the method of amplification according to the invention may advantageously be used to determine the phenotype of the HBV strains present in a biological sample. The phenotype of a HBV strain is intended for the determination of the resistance of said strain to one or several antiviral drugs, in particular drugs interacting with HBV replication, *in vitro* and/or *in vivo.*

The amplicons obtained by the above method of preparing HBV genomic amplicons can be either directly or indirectly used for the phenotypic (i.e. determination of resistance to antiviral drugs) and replicative analysis of HBV. Advantageously, the amplicons have been cloned and amplified and/or selected for viral sub-populations according to step (e) of the method of preparing HBV genomic amplicons.

The method of phenotyping according to the invention relies on the determination and the comparison of the replicative capacity of the population, or of a sub-population, of HBV strains present in the biological sample obtained from the patient, in the presence or in the absence of drugs influencing viral replication.

The method involves transfection of cells that support HBV replication with the HBV genomic amplicons according to the invention, either directly obtained at step (d) of the method of preparing HBV amplicons or further cloned, amplified and/or selected (step (e)). The transfected cells are cultured with, or without, agents capable of influencing viral replication. The viral DNA and/or proteins produced by the transfected cells, that have been cultured with, or without, said agents, may be quantified to determine the phenotypic resistance of HBV strains present in the biological sample. The replicative capacity of these strains may be determined by quantification of the viral DNA and/or proteins produced by the transfected cells that have been cultured without agents capable of interfering with HBV replication.

Thus the invention relates to a method of determining replicative capacity of a population, or sub-population, of HBV strains likely to be present in a biological sample of a patient, and/or determining the resistance of said strains to agents interfering with viral replication, which method comprises the steps consisting of:
a) transfecting the amplicons obtained by the method of preparing HBV genomic amplicons, as described above, into cells of human origin that are permissive to HBV replication, optionally in the presence of an agent capable of interfering with HBV replication;
b) optionally treating said cells with DNAse;
c) culturing said cells, optionally with increasing concentrations of an agent capable of interfering with HBV replication,
d) extracting the viral DNA and/or proteins produced by the cultured cells; and
e) quantifying the extracted viral DNA and/or proteins.

Step a) consists of transfecting the amplicon obtained by the method of preparing HBV genomic amplicons. Said transfection step may be carried out in the presence of an agent (or increasing concentrations of said agent) capable of interfering with HBV replication, as defined below. The term "transfection" means the introduction of a foreign nucleic acid (e.g. the amplicons or amplified and/or selected amplicons) into a host cell. A "host cell" means a cell that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described infra. As used herein, a host cell is a cell permissive to HBV replication. Said cells permissive to HBV replication may be human hepatoma cell lines, preferably the HuH7 cell line (Nakabayashi et al., 1982), HepG2 cells (ATCC HB8065), HepaRG cells (Gripon et al., 2002) or primary hepatocytes. Transfection may be carried out by means of the various techniques readily known by the one skilled in the art. For instance, the use of liposomal forms of the Fugen type (Roche) may be used for the transfection. The cells may be placed in culture and transfected 24 hours after distribution of the cells in conditions recommended by the transfection reagent manufacturer. Other products, such as lipofectamine or calcium chloride, or other methods such as electroporation technique, readily known by the one skilled in the art, may be used for transfection.

Advantageously, the method of phenotyping / assessing replicative capacity according to the invention involves a post-transfection step of dissociation and treatment with DNAse which makes it possible then to implement an assay in a high throughput phenotyping format. This treatment makes it possible to eliminate the majority of the DNA which was used for the transfection and which did not enter into the cells. This DNA would interfere during the final quantification step of the method, in particular where real time PCR is used. The treatment step (b) may be carried out approximately 60 hours after the transfecting step (a). Where the transfected cells are adherent cells, they are preferably treated with an agent which permits cell dissociation, such as trypsine, according to conventional procedures. Cells are pelleted, and treated with DNAse in order to eliminate the residual DNA from the transfection step. The step (b), especially where the cells are dissociated, also makes it possible to make the cells homogeneous and individualized for distribution in a culture format suitable for carrying out an assay in high throughput format.

The transfected cells may be then treated with agents capable of interfering with viral replication, i.e. inhibiting or blocking viral replication. Where transfection in step (a) has been carried out in the presence of an agent capable of interfering with viral replication, or increasing concentrations of said agent, the same agent, and where appropriate ranges of concentrations, are used for the culturing step (c). Preferably said agent is at least capable of interfering with HBV replication *in vitro.* These agents may be medicinal products used clinically or drugs undergoing preclinical or clinical development or more generally any drug likely to influence HBV replication. The agent capable of interfering with HBV replication may be selected from the group consisting of lamivudine, adefovir, entecavir, emtricitabine, clevudine, telbivudine, tenofovir, elvucitabine, any combination thereof, and any combination of these above agents with other drug likely to interfere with HBV replication.

The treatment begins with the addition to the medium containing the cells of a concentration range of the agent to which HBV resistance is to be studied. The treatment with the agent is continued by regularly changing the medium containing the concentration range studied. The minimum treatment period is generally three days.

The viral genetic material and/or the viral proteins produced during the culturing step (c) are then extracted from the cells or from the culture supernatants. The viral DNA and/or extraction step (d) may be carried out according to any method known by the one skilled in the art.

Viral proteins may be quantified by any method known by the one skilled in the art, such as ELISA, RIA, etc. In particular, the replicative capacity and/or phenotype of a population or sub-population of HBV strains may be assessed by quantifying HBe antigen and/or HBs antigen. Commercial kits for quantifying HBe and HBs antigens are available.

The method of phenotyping according to the invention may advantageously involve a step of extraction of the viral DNA in a format allowing high throughput analysis for phenotyping or replicative assay.

A rapid and simple method for extracting HBV DNA has been developed by the inventors. It can be carried out whatever the culture format used and whatever the HBV production strategy used. This extracting method makes it possible to obtain viral DNA extraction on any cell type, lines constitutively expressing HBV, lines transfected with a construct expressing HBV, whether it is produced by virtue of its own promoter or whether it is regulated by a strong promoter. This technique is especially suitable for real time PCR quantification in a high throughput format such as the 96-well format. The extraction method provided by the invention is carried out entirely in liquid phase, directly in the container used during the culturing, and the extract obtained may be directly quantifiable by real time PCR. Accordingly, this method is particularly advantageous where real time PCR is to be used for viral DNA quantification.

This extraction method comprises the steps consisting of:
1) dissociating and digesting the cells or the culture supernatants with DNAse and RNAse;
2) alkalinizing the digested cells or supernatants;
3) neutralizing, and optionally diluting for carrying out real time PCR directly.

The extraction is preferably carried out under conditions allowing to destroy all the DNAs and RNAs, with the exception of the encapsidated (and therefore protected in the viral capsid) HBV DNA which comes directly from the replication of the virus. For instance the digestion step (1) may be carried out by washing the cells with PBS and adding the extraction buffer (25 mM Tris, 0.5 mM CaCl₂, 2.5 mM MgCl₂, pH 8, 0.4 mg/ml DNAse, 0.4 mg/ml RNAse, 1% NP40), i.e. 50 µl per 30 mm² (96-well format). The treatment with DNAse and RNAse may last one hour at 37°C.

The viral DNA produced during the culturing, and which is protected in the viral capsids, is extracted in step (2) by denaturing the viral capsids by means of alkalinization of the extraction buffer, for instance by addition of NaOH, i.e. 15 µl of a 0.4 M solution per 50 µl of extraction buffer, preferably to reach a pH greater than 12 (Newman et al., 2003). This denaturation may be carried out for instance at 37°C for 30 min.

The denaturation step (2) may be stopped by adding a neutralizing buffer, for instance 15 µl of 1 M Tris at pH 7;

The method of extraction herein described is very rapid (less than 2 hours) and does not require any particular (rare or expensive) material or product.

According to an embodiment, the replicative capacity of the population or of a sub-population of HBV strains present in the biological sample of the patient is determined by quantifying the extracted viral DNA produced by the cultured cells in the absence of said agent capable of interfering with HBV replication. Accordingly, the quantity of viral DNA produced by the cultured cells in the absence of said agent capable of interfering with HBV replication is indicative of the replicative capacity of said population or sub-population of HBV strains present in said biological sample.

According to another embodiment, the resistance or the sensibility of the population or of a sub-population of HBV strains present in the biological sample of the patient to agents influencing viral replication is further determined by quantifying and comparing the extracted viral DNA produced by the cultured cells in the presence and in the absence of increasing concentrations of said agent capable of interfering with HBV replication.

Preferably, the replicative capacity is compared with that of a reference strain known to lack resistance to said agents influencing viral replication. Such reference strains are readily known by the one skilled in the art. Examples of appropriate reference strains include any genotype D, serotype ayw with wild type genotype for all known mutations or any virus found in untreated patients. For instance, the measures of the viral DNA produced by the cells transfected with the amplicons make it possible to determine the IC₅₀ value of the agent capable of interfering with HBV replication on the production of viral DNA by the population or sub-population of HBV strains present in the biological sample. This IC₅₀ value may be determined by identifying the concentration at which said agent interfering with HBV replication inhibits by 50 % HBV replication. This may be achieved by determining the concentration at which the quantity of viral DNA and/or protein is decreased by 50 %.

This IC₅₀ value may be compared with that measured on said cells of human origin that are permissive to HBV replication but that have been transfected with a reference virus lacking resistance to the agent capable of interfering with HBV replication. An increased IC₅₀ value measured with the cells transfected with amplicons according to the invention, as compared to the IC₅₀ value measured with the cells transfected with the reference virus, is indicative of the presence of a population or a sub-population of HBV strains, in the biological sample of the patient, which is resistant to the agent capable of interfering with HBV replication. This phenotypic resistance analysis may be repeated with several antiviral drugs to determine a pattern of resistance of the HBV strains the patient is infected with. The knowledge of the phenotypic resistance has clinical implications since it makes it possible to adapt the treatment of the patient in view of the pattern of resistance exhibited.

The quantification of the viral DNA may be carried out according to any techniques well known by the one skilled in the art to quantify this virus. However, DNA quantitation is preferably performed using real time PCR, with primers that allow specific quantification of HBV whatever its genotype.

Advantageously the pairs of primers for quantification by a real time PCR technique enable to quantify DNA of all known HBV genotypes (from A to H). Such primers preferably have the following characteristics:
(i) they have 100% homology with a consensus sequence representing 90% of the whole HBV genomes independently of the genotype ;
(ii) they are homologous, to within one base, to 98% of the whole HBV genomes ; and
(iii) they allow real time PCR quantification, i.e.:
   (1) they allow the amplification of amplicons less than 200 base pairs in size ; and
   (2) they do not form dimers of primers or of secondary DNA structures which may interfere with the quantification.

According to a preferred embodiment, the pair of primers used for DNA quantification by real time PCR is selected from the group consisting of SEQ ID No.46 and SEQ ID No.47, SEQ ID No.46 and SEQ ID No.48, and SEQ ID No.49 and SEQ ID No.50, as defined below:

The position of the first base is indicated according to the position numbering of SEQ ID No.1:
Pair A: 
Sequence sense : 5'-(254)TCG TGG TGG ACT TCT CTC A-3' (SEQ ID No.46).
Sequence antisense : 5'-(393)AAA CGC CGC AGA CAC ATC-3' (SEQ ID No.47).
Pair B 
Sequence sense : 5'- -(254)TCG TGG TGG ACT TCT CTC A -3' (SEQ ID No.46).
Sequence antisense : 5'- (427)GAG GCA TAG CAG CAG GAT-3' (SEQ ID No.48).
Pair C

Sequence sense 5'- (374)TGG ATG TGT CTG CGG CGT T-3' (SEQ ID No.49).
Sequence antisens : 5'- (477)GGA CAA ACG GGC AAC ATA-3' (SEQ ID No.50).

Still preferably, the primers have the following characteristics:
(i) they are homologous, to within one base, to 98% of the whole HBV genomes;
(ii) they are located in the HBV genome within the Enhancer I region, i.e. in the region between nucleotides 1175 and 1285 of the HBV genome as shown in SEQ ID No.1; and
(iii) they allow real time PCR quantification, i.e.:
   (1) they allow the amplification of amplicons less than 100 base pairs in size; and
   (2) they do not form dimers of primers or of secondary DNA structures which may interfere with the quantification.

According to a preferred embodiment, these primers have the sequences as defined below:

Sequence sense 5'-(1186)GCT GAC GCA ACC CCC ACT-3' (SEQ ID
No.51) ;

Sequence antisense 5'- (1283)AGG AGT TCC GCA GTA TGG-3' (SEQ ID
No.52).

The quantity of DNA is measured after each cycle of amplification, using conventional DNA dyes, for instance using SYBR Green (Molecular Probes) which becomes fluorescent upon binding to dsDNA.

The invention further provides a kit for quantifying HBV nucleic acid, which kit comprises :
- at least a pair of primers, as defined above;
- means for amplifying a HBV nucleic acid.

According to a preferred embodiment, said a pair of primers is selected from the group consisting of SEQ ID No.46 and SEQ ID No.47, SEQ ID No.46 and SEQ ID No.48, SEQ ID No.49 and SEQ ID No.50, and SEQ ID No.51 and SEQ ID No.52.

Preferably, the means for amplifying HBV nucleic acid are means for amplification by real time Polymerase Chain Reaction.

The real time PCR quantification method presented here is capable of quantifying all the HBV genotypes with the same efficiency. This real time PCR quantification lasts approximately one hour. The 50% inhibitory concentrations (IC₅₀) are calculated during this quantification with respect to the replication obtained without drug. The level of replication without drug corresponds to the replicative capacity of the viral population of the patient *in vitro* and is compared to that of a reference wild-type strain.

By virtue of these novel developments, it is now possible to perform, in a high throughput format, the extraction of virions and the quantification thereof by real time PCR in less than 3 hours.

The technology previously used for quantifying viral production (Southern blotting) required more than 2 weeks to obtain the sufficient levels of HBV replication in culture, with a restricted number of samples.

The invention will be further illustrated in view of the following figures and examples.

### FIGURES

Figure 1 is a diagrammatic representation of the method of preparing HBV genomic amplicons according to the invention where the pair of primers hybridizes in any HBV region outside the gap region.
Figure 2 is a diagrammatic representation of the method of preparing HBV genomic amplicons according to the invention where the pair of primers hybridizes in HBV gap region.
Figure 3 displays the increase in replication levels with the amplification of the method of the invention, by comparison with the Günther method (using the Sap I site) in a 12-well format and analysis by Southern blotting. The genotypes D, A and C originate from patients. Sap: Günther method. Nar: method of the invention with introduction of the Nar I site. Arrow and box: relaxed circular form of intracapsid HBV.

### EXAMPLES

### Example 1: Efficiency of amplification of the clinical isolate

The method of the invention enables efficient PCR amplification.

The whole genome amplification protocol is preferably carried out with the Roche High Fidelity enzyme and consists of the following steps:
+ Hot-Start step: 3 min at 94°C; with addition of the enzyme after 1 min ;
+ 40 cycles :
   - 40 sec at 94°C
   - 1 min 30 sec at 55°C [first 10 cycles] and at 60°C [other 10 cycles] then at 62°C [other 20 cycles]
   - 3 min [+ 2 min after each 10 cycles]) at 68°C ;
+ a step of 10 min at 68°C.

This protocol may be performed with the primers specific for the Nar I site (sense sequence SEQ ID No.44, and antisense sequence SEQ ID No.45).

### Example 2: Better level of viral replication

The method of the invention gives a better level of viral replication than strategy 1 (Gunther).

The method for amplifying whole HBV genomes according to the incvention, as exemplified in example 1 with introduction of the Nar I site, makes it possible to obtain the overall viral population present in a patient without creating a chimeric genome (unlike strategy 2). It was verified that the introduction of this Nar I site did not engender any amino acid variation in the only protein involved in this region: the X protein. In addition, the genomes amplified by the strategy according to the invention normally produce the viral proteins in the culture supernatant, for instance the HBs and HBe antigens, assayed using commercial kits (Monolisa Ag HBe and HBs from BIO-RAD Laboratories).

Once the amplification had been carried out, after digestion with the Nar I enzyme and transfection, the replication levels obtained were studied and it was observed that, with the construct produced with the method according to the invention, the replication levels obtained are higher than those obtained with the Günther strategy.

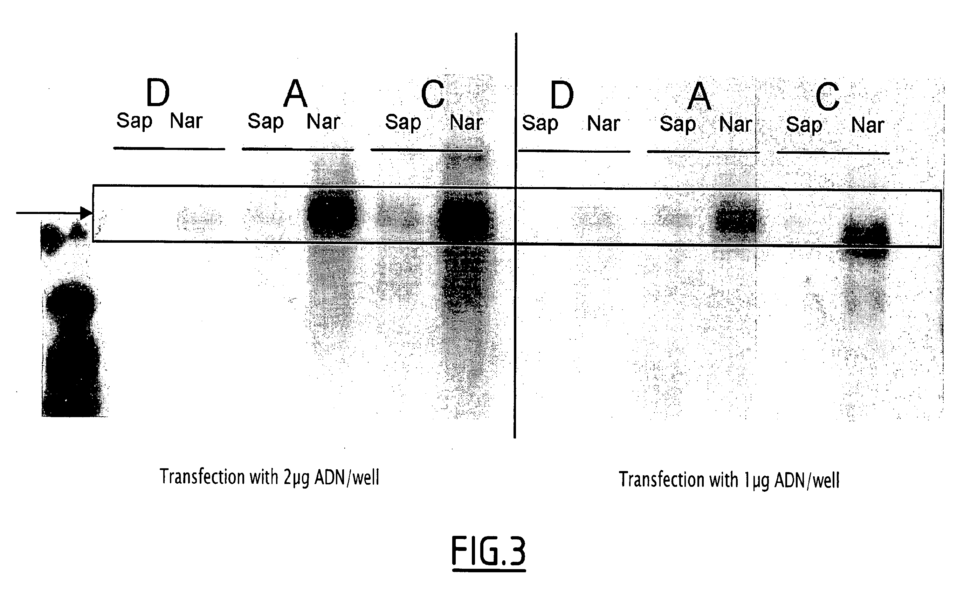

It was confirmed, using sera from patients of various genotypes (A, C and D), that the replication levels are systematically higher with the whole genomes amplified with the strategy of the invention than with those amplified with the Günther method (Figure 3) in a format consisting of conventional culture and visualization by Southern blotting.

This increase in replication was confirmed using the viral replication quantification method of the invention (high throughput format and real time PCR), and also correlates with a systematic increase in the viral proteins produced and released into the culture supernatants (Table I).

### Table I

Levels of replication and of excretion of the HBe antigen of genotypes A, C and D in a high throughput format and visualization of the viral production by real time PCR of the method of the invention

| Genotype | Method | Replication copy/reaction | Increase copy/reaction | HBe antigen (OD) | Increase HBe antigen |
|---|---|---|---|---|---|
| A | Genome | 1.48^{E}+03 | 4.8 | 0.351 | 1.62 |
| | Nar I | | | | |
| | Günther | 3.07^{E}+02 | | 0.216 | |
| | Sap I | | | | |
| C | Genome | 3.83^{E}+03 | 2.48 | 0.311 | 1.10 |
| | Nar I | | | | |
| | Günther | 1.54^{E}+03 | | 0.281 | |
| | Sap I | | | | |
| D | Genome | 4.12^{E}+02 | 7.01 | 0.378 | 1.78 |
| | Nar I | | | | |
| | Günther | 5.87^{E}+01 | | 0.212 | |
| | Sap I | | | | |

The HBe antigen, which correlates directly with the replication, is systematically expressed to a greater degree with the novel strategy. For this antigen, there is a risk of encountering a problem of detection with patients who are clinically HBe negative as with patient chronically infected by the genotype G.

The method for amplifying a whole HBV genome according to the invention therefore keeps the advantages of the Günther method, i.e. amplification of all the viral genomes present in the patients, without creating chimeric viruses but especially with better replication of the genomes obtained.

The replication with the novel strategy is 2.5 (genotype D) to 7 times (genotype C) greater than that obtained with the conventional Günther strategy.

In addition to this better replication, this amplification strategy, as exemplified with the Nar I site, has the advantage of introducing a unique cleavage site which is 3 times less frequent than the Sap I site used by Günther, in the published HBV genomes (5 cleavages out of 971 sequences published for the Nar I enzyme against 16 for the Sap I enzyme). This advantage makes it possible to study virtually all the viral strains of HBV and to greatly limit the risk of obtaining HBV genomes with internal Nar I sites (0.5% of the genomes, against 1.5% for the Sap I site).

### Example 3: High throughput phenotyping assay genotypic analysis and replication capability quantification.

The method of the invention makes it possible to carry out a phenotyping assay that is more rapid, more sensitive, more reproducible and easier to adapt to a large volume of samples from patients. In the same time, the genotypic analysis and the evaluation of the replication capability of the quasi-species found in the serum of patients can be performed on the same amplicon.

By virtue of this novel method for amplifying all the viral genomes present in a patient and the various technological advances which make it possible to carry out the culture and extraction steps in a format suitable for a high throughput phenotypic analysis, an analysis on 6 samples originating from the therapeutic monitoring of various patients was performed.

The data relating to the patients, viral loads, ongoing treatment, therapeutic monitoring, were not known at the beginning of the experiment.

In addition to the 6 samples analysed, a control was included. This control is a genome of the reference HBV, genotype D, having an *ayw* serotype and a wild-type phenotype for all the drugs studied. This genome was amplified by the same technique as the samples analysed using a plasmid.

### Experimental procedure:

- The DNA from 1 ml of serum was extracted using the QIAamp Ultrasens Virus kit (Qiagen), and was taken up in 60 µl of water.
- An amplification of the whole genomes by the method of the invention including the Nar I site was carried out and the amplicons obtained were purified by means of a silica column (Qiagen kit, Qiaquick purification kit).

Subgenomic PCR were performed on these amplicons to carry out the INNOLiPA analysis (Lok et al., 2002) which gives the profile of known mutations confering resistance observed during treatment with Lamivudine and Adefovir. In addition, for one sample (N°19) direct sequencing was performed on the same amplicon used for the phenotyping and the genotyping (INNOLiPA) analysis to look for the N236T mutation associated to the resistance to Adefovir (Angus et al., 2003; Villeneuve et al., 2003). For INNOLiPA analysis, the mutations are indicated by reference to the amino acid sequence of HBV polymerase.

The amplicons were then digested with the Nar I enzyme and purified by the same method as above.

These amplicons were transfected, in a culture format comprising a 25 cm² flask seeded the day before at a density of 5.2 × 10⁴ cells/cm2, with 200 to 400 ng of amplicon/cm².

After 60 hours, the cells were trypsinized and then treated with DNAse. After trypsinization, the cells were taken up in culture medium supplemented 50/50 with the two-times concentrated DNAse buffer (50 mM Tris, 1 mM CaCl₂, 5 mM MgCl₂, pH 8, 400µg/ml of DNAse I) containing DNAse I, and incubated for 45 min at 37°. After pelleting, the cells were taken up in culture medium and distributed into 96-well culture plates coated (BioCoat) with collagen at a density of 6 × 10⁴ cells/cm² in a volume corresponding to half the final volume used for the treatment with the drugs to be tested. This volume was completed with the 2-times concentration range of the drugs studied (Lamivudine and Adefovir)

The ranges used were from 100 to 0.01 µM for Lamivudine and from 100 to 6.25 µM for Adefovir. These cultures were treated with these dilutions for 4 days.

At the end of these 4 days of treatment, the viral DNA was extracted.

This step was carried out after washing the cells with PBS and adding the extraction buffer, i.e. 50 µl per 30 mm² (96-well format) (25 mM Tris, 0.5 mM CaCl₂, 2.5 mM MgCl₂, pH 8, 0.4 mg/ml DNAse, 0.4 mg/ml RNAse, 1% NP40). The lysis lasted one hour at 37°C with agitation on a Heidolph Titramax 1000 plate mixer at a rotation rate of about 1050 rpm.

After this step of lysis and of digestion with DNAse and RNAse, the viral DNA produced during the culturing remains protected in the viral capsids. This DNA was then extracted by denaturation of the viral capsids by means of alkalinization of the extraction buffer by addition of NaOH, i.e. 15 µl of a 0.4M solution per 50 µl of extraction buffer. This denaturation was carried out at 37°C for 30 min under the same agitation conditions as during the treatment with DNAse, i.e. on a Heidolph Titramax 1000 plate mixer at a rotation rate of about 1050 rpm.

This denaturation step was then stopped by adding a neutralizing buffer, 15 µl of 1 M Tris at pH 7, and the solution thus obtained was subjected to a dilution before quantification by real time PCR. The final dilution is 1/5 relative to the initial volume of extraction buffer, i.e. an addition of 170 µl of water in a 96-well format.

The viral DNA extracted was then quantified by means of real time PCR quantification method.

This HBV production quantification step can be directly carried out after the preceding step, without any need to purify or concentrate the cell lysates to be assayed. The real time PCR can be carried out in the same format as the culture format, i.e. a 96-well format on the Biorad MyiQ device, using the real time kit and the consumables suitable for this device, i.e. the iQ^{™} Sybr® Green Supermix two-times concentrated PCR mix. The real time PCR protocol used is a protocol of at most 40 cycles of two steps after activation of the polymerase by hot start, i.e.: 95°C 3 min (Hot Start and calibration of the device), 40 cycles of denaturation: 15 seconds at 95°C and of annealing 20 seconds at 63°C (quantification step), and a final extension of one minute at 63°C. The final reaction volume is 25 µl consisting of 12.5 µl of iQ^{™} Sybr® Green Supermix, 1 µl of each primer (sequences SEQ ID No.51 and SEQ ID No.52) at 7.5 µM (i.e. a final concentration of 300 nM), 5.5 µl of water and 5 µl of test sample.

The total duration of this quantification is approximately one hour.

For each plate assayed, a standard curve of 10⁶ to 10² HBV copies/reaction was quantified in parallel. This standard curve is produced with a cloned HBV genome (ayw, genotype D) of known concentration determined by optical density assay.

Under these conditions, the efficiency of the real time amplification is always between 95 and 105% and the standard curve has a linearity coefficient that is always greater than 0.99. This linearity was, moreover, validated over a concentration range of 10⁸ to 10 copies per reaction.

Each point was analysed by real time PCR in quadruplicate.

The total duration of the analysis, from the extraction of the DNA from the serum up until the issuing of the results, was 10 days.

### Results

The IC₅₀ values of these samples for Lamivudine and for Adefovir (Table 2) were compared with the IC₅₀ values obtained with the reference wild-type virus (genotype D, ayw, without known mutation for these drugs), and an in vitro resistance was determined.

After this analysis, the clinical data for these samples were obtained and compared with the results of the *in vitro* phenotyping (Table 2). A genotypic analysis was carried out on these same samples by INNOLiPA. For this analysis, subgenomic amplicons were produced and hybridized as defined by Innogenetics.

Finally, the estimation of the replication capability was defined as the untreated point of the phenotyping analysis, i.e. the HBV viral DNA produced by the transfected cells cultured in absence of Lamiduvine or Adefovir. This *in vitro* estimation of the replication capability is very close to the *in vivo* situation since the replication of the viral genomes *in vitro* is driven by the natural HBV promoter. The result of the replication capability is summarized in Table 2.

**Table 2 : Comparison of the clinical data and of in vitro phenotyping**

| | Referent virus | N° 3 | N° 5 | N° 7 | N° 17 | N° 19 | N° 21 |
|---|---|---|---|---|---|---|---|
| Monitoring point | | D0 3TC | D0 3TC | M18 3TC | M24 3TC | M24 3TC M3 PMEA | D0 3TC |
| Viremia bDNA | | 3803 | 367.3 | 41.1 | < 0.7 | 546.8 | 28.2 |
| Clinical phenotype | | Naïve | Naïve | Viral escape? | Viral escape? | Viral escape? | Naïve |
| IC 50 Lamivudine µM | 0.1 | 0.7 | 0.2 | >100 | >100 | 40 | 0.14 |
| Increase in resistance to Lamivudine | / | 7 | 2 | >1000 | >1000 | 400 | 1.4 |
| *in vitro* Lamivudine phenotype | | S | S | R | R | R | S |
| IC₅₀ Adefovir µM | 8 | 9 | 8 | 13.5 | 14 | 52 | 14.5 |
| Increase in resistance to Adefovir | / | 1.1 | 1 | 1.7 | 1.75 | 6.5 | 1.8 |
| *in vitro* Adefovir phenotype | | S | S | S | S | R | S |
| Replication capability Copy / reaction | | 2.10³ | 1.4 10⁴ | 1.6 10³ | 4.5 10³ | 1.4 10³ | 7.510² |
| INNO LiPA genotypes | | wt | wt | L80V M204I | L180M ± M204I | L180M A181V | wt |

| | | | | | | | |
|---|---|---|---|---|---|---|---|
| D0 = day 0 | | | | | | | |
| M = month | | | | | | | |
| 3TC = Lamivudine | | | | | | | |
| PMEA = Adefovir | | | | | | | |
| bDNA = quantification of viral load in serum from patient by the bDNA system of Bayer. | | | | | | | |

INNO LiPA Genotype = mutation profile find by the INNO LiPA system of Innogenetic on HBV genomic amplicon for the known mutation for Lamivudine L180M, M204V, M204I or for Adefovir N236T or A181V. For sample N°17, a mixed population of mutants L180M and double mutants L180M + M204I was found.

The data obtained by means of the phenotyping assay according to the invention are in accordance with the clinical observations in these various patients and with the INNOLiPA genotyping data. It should be noted that this agreement between genotyping and phenotyping shows the advantage of carrying out the two analyses using a common amplicon.

For point 19, which is doubly resistant to Lamivudine and to Adefovir, the INNOLiPA analysis shows the presence of a mutation described as being associated with a decrease in susceptibility to Adefovir: A181V (Bartholomeusz et al., 2004) although the mutation N236T is not present. The search for the latter mutation by direct sequencing was negative. Furthermore, in this patient, a lower resistance to Lamivudine is associated with the L180M mutation.

In conclusion, the method of amplifying HBV genomes and the format for carrying out the phenotyping assay described herein allow to rapidly (less than 10 days) obtain, for a large number of samples, a phenotypic analysis of the viral population present in a patient.

This analysed population reflects the quasi-specie found in the patient, without the creation of any chimeric virus during the amplification, which guarantees that all the mutations present on a genome are taken into account for the phenotypic analysis.

This analysis can be carried out with the drugs currently clinically available or in preclinical development, and may be carried out with all the drugs available at a given time in the future.

Furthermore, it should be noted that the amplicon used for the phenotypic analysis can be directly used for a genotypic analysis, as was done for the trials herein described.

### REFERENCES

AASLD. (2004) Abstracts of the 55th Annual Meeting of the American Association for the Study of Liver Diseases. Boston, Massachusetts, USA, October 29-November 2004. Hepatology 40(4 Suppl 1), 162A-771A.
Allen, M.I., Deslauriers, M., Andrews, C.W., Tipples, G.A., Walters, K.A., Tyrrell, D.L., Brown, N. and Condreay, L.D. (1998) Identification and characterization of mutations in hepatitis B virus resistant to lamivudine. Lamivudine Clinical Investigation Group. Hepatology 27(6), 1670-7.
Angus, P., Vaughan, R., Xiong, S., Yang, H., Delaney, W., Gibbs, C., Brosgart, C., Colledge, D., Edwards, R., Ayres, A., Bartholomeusz, A. and Locarnini, S. (2003) Resistance to adefovir dipivoxil therapy associated with the selection of a novel mutation in the HBV polymerase. Gastroenterology 125(2), 292-7.
Bartholomeusz, A., Tehan, B.G. and Chalmers, D.K. (2004) Comparisons of the HBV and HIV polymerase, and antiviral resistance mutations. Antivir Ther 9(2), 149-60.
Bock, C.T., Tillmann, H.L., Torresi, J., Klempnauer, J., Locarnini, S., Manns, M.P. and Trautwein, C. (2002) Selection of hepatitis B virus polymerase mutants with enhanced replication by lamivudine treatment after liver transplantation. Gastroenterology 122(2), 264-73.
Durantel, D., Carrouee-Durantel, S., Werle-Lapostolle, B., Brunelle, M.N., Pichoud, C., Trepo, C. and Zoulim, F. (2004) A new strategy for studying in vitro the drug susceptibility of clinical isolates of human hepatitis B virus. Hepatology 40(4), 855.
Gripon, P., Rumin, S., Urban, S., Le Seyec, J., Glaise, D., Cannie, I., Guyomard, C., Lucas, J., Trepo, C. and Guguen-Guillouzo, C. (2002) Infection of a human hepatoma cell line by hepatitis B virus. Proc Natl Acad Sci U S A 99(24), 15655-60.
Gunther, S., Li, B.C., Miska, S., Kruger, D.H., Meisel, H. and Will, H. (1995) A novel method for efficient amplification of whole hepatitis B virus genomes permits rapid functional analysis and reveals deletion mutants in immunosuppressed patients. J Virol 69(9), 5437-44.
Hall, T. (1999) BioEdit: a user-friendly biological sequence alignment editor and analysis program for Windows 95/98/NT. Nucl. Acids. Symp. Ser 41, 95-98.
ICAR, A. (2004) Abstracts of the 17th International Conference on Antiviral Research. Tucson, Arizona, USA. May 2-6, 2004. Antiviral Res 62(2), A27-90.
Lada, O., Benhamou, Y., Cahour, A., Katlama, C., Poynard, T. and Thibault, V. (2004) In vitro susceptibility of lamivudine-resistant hepatitis B virus to adefovir and tenofovir. Antivir Ther 9(3), 353-63.
Ladner, S.K., Otto, M.J., Barker, C.S., Zaifert, K., Wang, G.H., Guo, J.T., Seeger, C. and King, R.W. (1997) Inducible expression of human hepatitis B virus (HBV) in stably transfected hepatoblastoma cells: a novel system for screening potential inhibitors of HBV replication. Antimicrob Agents Chemother 41 (8), 1715-20.
Lizardi, P.M., Huang, X., Zhu, Z., Bray-Ward, P., Thomas, D.C. and Ward, D.C. (1998) Mutation detection and single-molecule counting using isothermal rolling-circle amplification. Nat Genet 19(3), 225-32.
Lok, A.S., Zoulim, F., Locarnini, S., Mangia, A., Niro, G., Decraemer, H., Maertens, G., Hulstaert, F., De Vreese, K. and Sablon, E. (2002) Monitoring drug resistance in chronic hepatitis B virus (HBV)-infected patients during lamivudine therapy: evaluation of performance of INNO-LiPA HBV DR assay. J Clin Microbiol 40(10), 3729-34.
Nakabayashi, H., Taketa, K., Miyano, K., Yamane, T. and Sato, J. (1982) Growth of human hepatoma cells lines with differentiated functions in chemically defined medium. Cancer Res 42(9), 3858-63.
Newman, M., Suk, F.M., Cajimat, M., Chua, P.K. and Shih, C. (2003) Stability and morphology comparisons of self-assembled virus-like particles from wild-type and mutant human hepatitis B virus capsid proteins. J Virol 77(24), 12950-60.
Norder, H., Courouce, A.M. and Magnius, L.O. (1994) Complete genomes, phylogenetic relatedness, and structural proteins of six strains of the hepatitis B virus, four of which represent two new genotypes. Virology 198(2), 489-503.
Pichoud, C., Seigneres, B., Wang, Z., Trepo, C. and Zoulim, F. (1999) Transient selection of a hepatitis B virus polymerase gene mutant associated with a decreased replication capacity and famciclovir resistance. Hepatology 29(1), 230-7.
Saiki, R.K., Gelfand, D.H., Stoffel, S., Scharf, S.J., Higuchi, R., Horn, G.T., Mullis, K.B. and Erlich, H.A. (1988) Primer-directed enzymatic amplification of DNA with a thermostable DNA polymerase. Science 239(4839), 487-91.
Seigneres, B., Pichoud, C., Martin, P., Furman, P., Trepo, C. and Zoulim, F. (2002) Inhibitory activity of dioxolane purine analogs on wild-type and lamivudine-resistant mutants of hepadnaviruses. Hepatology 36(3), 710-22.
Sells, M.A., Chen, M.L. and Acs, G. (1987) Production of hepatitis B virus particles in Hep G2 cells transfected with cloned hepatitis B virus DNA. Proc Natl Acad Sci U S A 84(4), 1005-9.
Tenney, D.J., Levine, S.M., Rose, R.E., Walsh, A.W., Weinheimer, S.P., Discotto, L., Plym, M., Pokornowski, K., Yu, C.F., Angus, P., Ayres, A., Bartholomeusz, A., Sievert, W., Thompson, G., Warner, N., Locarnini, S. and Colonno, R.J. (2004) Clinical emergence of entecavir-resistant hepatitis B virus requires additional substitutions in virus already resistant to Lamivudine. Antimicrob Agents Chemother 48(9), 3498-507.
Villeneuve, J.P., Durantel, D., Durantel, S., Westland, C., Xiong, S., Brosgart, C.L., Gibbs, C.S., Parvaz, P., Werle, B., Trepo, C. and Zoulim, F. (2003) Selection of a hepatitis B virus strain resistant to adefovir in a liver transplantation patient. J Hepatol 39(6), 1085-9.
Yang, H., Westland, C., Xiong, S. and Delaney, W.E.t. (2004) In vitro antiviral susceptibility of full-length clinical hepatitis B virus isolates cloned with a novel expression vector. Antiviral Res 61(1), 27-36.
Zhang, D.Y., Brandwein, M., Hsuih, T.C. and Li, H. (1998) Amplification of target-specific, ligation-dependent circular probe. Gene 211 (2), 277-85.

## Claims

1. A method for preparing a HBV genomic amplicon from a biological sample of a patient, which method comprises the steps consisting of :
a) extracting HBV nucleic acid from the biological sample;
b) amplifying the HBV nucleic acid with a pair of primers that are selected to obtain amplicons comprising the entire HBV genome, and that comprise a copy of a restriction site that is not present or is present only once in a consensus sequence of HBV genome of all genotypes, wherein said restriction site is cut by a restriction enzyme within the recognition sequence for said restriction enzyme;
c) digesting the amplicons with the restriction enzyme which cuts said restriction site;
and
d) purifying the digested amplicons.

2. The method according to claim 1, wherein the primers comprise a nucleotide sequence complementary to the HBV gap region defined by nucleotide positions 1800 to 1850 of SEQ ID No1.

3. The method according to claim 1; wherein said restriction site is not present in the consensus sequence of HBV genome of all genotypes and wherein said restriction site is substantially absent of all HBV sequences and/or does not introduce more than 3 nucleotide changes in the HBV genomic sequence.

4. The method according to any of claims 1 to 3, wherein said restriction site is selected from the group consisting of BssH II, Cla I, Mlu I, Nhe I, Not I, Pvul, Rca I, Sun I (BsiW I), and Nar I.

5. The method according to any of claims 2 to 4, wherein the primers comprise the nucleotide sequence of the Nar I restriction site (SEQ ID No.41) and a nucleotide sequence complementary to the HBV gap region.

6. The method according to any of claims 1 to 5, wherein the pair of primers is selected from the group consisting of :
SEQ ID No.42 and SEQ ID No.43,
SEQ ID No.44 and SEQ ID No.45,
SEQ ID No.11 and SEQ ID No.12,
SEQ ID No.14 and SEQ ID No.15,
SEQ ID No.18 and SEQ ID No.19,
SEQ ID No.22 and SEQ ID No.23,
SEQ ID No.26 and SEQ ID No.27,
SEQ ID No. 30 and SEQ ID No.31,
SEQ ID No.34 and SEQ ID No.35, and
SEQ ID No.38 and SEQ ID No.39.

7. The method according to any of claims 1 to 6, wherein the pair of primers is SEQ ID No.42 and SEQ ID No.43.

8. The method according to claim 7, wherein the pair of primers is SEQ ID No.44 and SEQ ID No.45.

9. The method according to any of claims 1 to 8, which comprises a further step (e) which consists of cloning the amplicons obtained in step (d) in a prokaryotic or eukaryotic vector, and amplifying the cloned amplicons and/or selecting amplicons of viral sub-populations, and extracting the amplicons amplified and/or selected from the vector.

10. A method of genotyping of HBV strains present in a biological sample of a patient, which method comprises the steps consisting of genotyping amplicons obtained by a method according to any of claims 1 to 9 .

11. A method of determining replicative capacity of a population or sub-population of HBV strains likely to be present in a biological sample of a patient, and/or determining the resistance of said strains to an agent capable of interfering with viral replication, which method comprises the steps consisting of:
a) transfecting the amplicons obtained by a method according to any of claims 1 to 9 into cells of human origin that are permissive to HBV replication, optionally in the presence of an agent capable of interfering with HBV replication;
b) optionally treating said cells with DNAse;
c) culturing said cells, optionally with increasing concentrations of an agent capable of interfering with HBV replication ;
d) extracting viral DNA and/or proteins produced by the cultured cells; and
e) quantifying the extracted viral DNA and/or proteins.

12. The method according to claim 11, wherein the replicative capacity of the population or of a sub-population of HBV strains present in the biological sample is determined by quantifying the extracted viral DNA produced by the cultured cells in the absence of said agent capable of interfering with HBV replication.

13. The method according to claim 11, wherein the resistance of the population or of a sub-population of HBV strains present in the biological sample to an agent capable of interfering with viral replication is determined by quantifying and comparing the extracted viral DNA produced by the cultured cells in the presence and in the absence of said agent capable of interfering with HBV replication.

14. The method according to claim 13, wherein IC₅₀ value of the agent capable of interfering with HBV replication on the production of viral DNA by said cells of human origin that are permissive to HBV replication and that have been transfected with said amplicons, is compared with the IC₅₀ measured on other said cells of human origin that are permissive to HBV replication but that have been transfected with a reference virus lacking resistance to the agent capable of interfering with HBV replication, and wherein an increased IC₅₀ value measured with the cells transfected with amplicons, as compared to the IC₅₀ value measured with the cells transfected with the reference virus, is indicative of the presence of a population or a sub-population of HBV strains which is resistant to the agent capable of interfering with HBV replication.

15. The method according to any of claims 11 to 14, wherein said agent capable of interfering with HBV replication *in vitro* is selected from the group consisting of lamivudine, adefovir, entecavir, emtricitabine, clevudine, telbivudine, tenofovir, elvucitabine, any combination thereof, and any combination of the above agents with other drug likely to interfere with HBV replication.

16. The method according to any of claims 11 to 15, wherein the extracted viral DNA is quantified by real time PCR using a pair of primers selected from the group consisting of :
SEQ ID No.51 and SEQ ID No.52,
SEQ ID No.46 and SEQ ID No.48
SEQ ID No.49 and SEQ ID No.50, and
SEQ ID No.46 and SEQ ID No.47.

17. A pair of primers selected from the group consisting of :
SEQ ID No.42 and SEQ ID No.43,
SEQ ID No.44 and SEQ ID No.45,
SEQ ID No.11 and SEQ ID No.12,
SEQ ID No.14 and SEQ ID No.15,
SEQ ID No.18 and SEQ ID No.19,
SEQ ID No.22 and SEQ ID No.23,
SEQ ID No.26 and SEQ ID No.27,
SEQ ID No. 30 and SEQ ID No.31,
SEQ ID No.34 and SEQ ID No.35, and
SEQ ID No.38 and SEQ ID No.39.

18. A pair of primers selected from the group consisting of :
SEQ ID No.51 and SEQ ID No.52,
SEQ ID No.46 and SEQ ID No.48
SEQ ID No.49 and SEQ ID No.50, and
SEQ ID No.46 and SEQ ID No.47.

19. A kit for preparing HBV genomic amplicons from a biological sample of a patient, which kit comprises :
- at least a pair of primers according to claim 17;
- means for amplifying a HBV nucleic acid.

20. A kit for quantifying HBV nucleic acid, which kit comprises:
- at least a pair of primers according to claim 18;
- means for amplifying a HBV nucleic acid.


SEQUENCE LISTING

<110> BioAlliance Pharma
INSERM

<120> METHOD OF GENOTYPING AND PHENOTYPING HEPATITIS B VIRUSES RESISTANT TO
ANTIVIRAL MOLECULES

<130> BET 05P0069

<160> 52

<170> PatentIn version 3.1

<210> 1
<211> 3215
<212> DNA
<213> Hepatitis B virus

<220>
<221> misc_feature
<222> (1)..(3215)
<223> HBV genomic sequence, genotype C, serotype adrq (Genbank X75665)

<400> 1
ctccacaaca ttccaacaag ctctgcagga tcccagagtc agggtccttt attttcctgc 60

tggtggctcc agttccggaa cagtaaaccc tgttccgact actgcctctc tcatttcgtc 120

aatcttctcg aggattgggg accctgtaac gaacatggag aacacaacat caggattcct 180

aggacccctg ctcgtgttac aggcggggtt tttcttgttg acaaaaatcc tcacaatacc 240

acagagtcta gactcgtggt ggacttctct caattttcta gggggagcac ccgtgtgtcc 300

tggccaaaat tcgcagtccc caacctccaa tcactcacca acctcttgtc ctccaatttg 360

tcctggctat cgctggatgt gtctgcggcg ttttatcatc ttcctcttca tcctgctgct 420

atgcctcatc ttcttgttgg ttcttctgga ctaccaaggt atgttgcccg tttgtcctct 480

acttccagga acatcaacta ccagcacggg accatgcaag acctgcacga ttcctgctca 540

aggaacctct atgtttccct catgttgctg tacaaaacct tcggacggaa actgcacttg 600

tattcccatc ccatcatcct gggctttcgt aagattccta tgggagtggg cctcagtccg 660

tttctcctgg ctcagtttac tagcgccatt tgttcagtgg ttcgtagggc tttcccccac 720

tgtttggctt tcagttatat ggatgatgtg gtattggggg ccaagtctgt acaacatctt 780

gagtcccttt atacctctat taccaatttt cttttgtctt tgggtataca tttaaaccct 840

aataaaacca aaagatgggg ctattccctt aacttcatgg gctatgtaat tggaagttgg 900

ggtaccttac cacaagaaca tattgtacta aaaatcacac aatgttttcg aaaacttcct 960

gttaataggc ctattgattg gaaagtgtgt caaagaattg tgggtctttt gggctttgct 1020

gcccctttta cacaatgtgg gtatcctgcc ttaatgccct tgtatgcctg tattcaagct 1080

aagcaggctt tcactttctc gccaacttat aaggcctttc tgtgtaaaca atatctgaac 1140

ctttaccccg ttgcccggca acggtctggt ctttgccaag tgtttgctga cgcaaccccc 1200
actggctggg gcttggccat gggccatcag cgcatgcgtg gaacctttgt ggctcctctg 1260
ccgatccata ctgcggaact cctagcggct tgttttgctc gcagccggtc tggagcaaac 1320
attatcggaa ccgacaactc tgtcgtcctc tctcggaaat acacatcctt tccatggctg 1380
ctcgggtgtg ctgccaactg gatcctacgc gggacgtcct ttgtttacgt cccgtcggcg 1440
ctgaatcccg cggacgaccc gtctcgcggc cgtttggggc tctaccgtcc ccttctttgt 1500
ctgcggttcc ggccaaccac ggggcgcacc tctctttacg cggtctcccc gtctgtgcct 1560
tctcatctgc cggaccgtgt gcacttcgct tcacctctgc acgtcgcatg gaaaccaccg 1620
tgaacgccca catggtcttg cccaaggtct tgcataagag aactcttgga ctctcagcaa 1680
tgtcaacgac cgaccttgag gcatatttca aagactgtgt gttcaaagac tgggaggagt 1740
tgggggagga ggttagatta aaggtctttg tactaggagg ctgtaggcat aaattggtct 1800
gcgcaccagc accatgcaac tttttcacct ctgcctaatc atctcatgtt catgtcctac 1860
tgttcaagcc tccaagctgt gccttgggtg gctttggggc atggacattg acccttataa 1920
agaatttgga gcttctgtgg agttactctc ttttttgcct tctgatttct ttccatctat 1980
tcgagacctc ctcgacaccg cctcagctct gtatcgggag gccttagagt ctccggagca 2040
ttgttcacct caccatacag cactcaggca agctgttctg tgttggggtg agttaatgaa 2100
tctggctacc tgggtgggaa gtaatttgga agacccagca tcaagagaat tggtagtcag 2160
ttatgtcaat gttaatatgg gcctaaaaat caggcaactg ttgtggtttc atatttcctg 2220
tcttactttt ggaagagaaa ctgttcttga gtacttggtg tcctttggag tgtggattcg 2280
cactcctccc gcttacagac caccaaatgc ccctatctta tcaacacttc cggaaactac 2340
tgttgttaga cgaagaggca ggtcccctag aagaagaact ccctcgcctc gcagacgaag 2400
gtctcaatcg ccgcgtcgca gaagatctca atctcgggaa tcccaatgtt agtatccctt 2460
ggactcataa ggtgggaaac tttactgggc tttattcttc tactgtacct gtctttaatc 2520
ctgaatggca aactccctct tttcctgaca ttcatttgca ggaggacatt attaatagat 2580
gtcaacaata tgtgggccct cttacagtta atgaaaaaag aagattaaaa ttaattatgc 2640
ctgctaggtt ttatcctaac cttactaaat atttgccctt agacaaaggc attaaacctt 2700
attatccaga acagacagtt aatcattact tcaaaactag gcattatttg catactctgt 2760
ggaaggctgg tagtctatat aagagagaaa ctacacgcag cgcctcattt tgtgggtcac 2820
catattcttg ggaacaagag ctacagcatg ggaggttggt cttcaaaacc tcggaaaggc 2880
atggggacga atctttcggt acccaatcct ctgggattct ttcccgatca ccagttggac 2940
cctgcgttcg gagccaactc aaacaatccc gattgggact tcaaccccaa caaggatcac 3000

tggccagagg caaatcaggt aggagcggga gcattcgggc cagggttcac cccaccacac 3060

ggaggtcttt tggggtggag ccctcaggcc cagggcatat tgacaacagt gccagcagct 3120

cctccttctg cctccaccaa tcggcagtca ggaagacagc ctacgcccat ctctccacct 3180

ctaagagaca gtcatcctca ggccatgcag tggaa 3215

<210> 2
<211> 3221
<212> DNA
<213> Artificial Sequence

<220>
<223> HBV genotype A consensus sequence

<400> 2
ttccacwgcc ttccaccaag ctctgcarga tcccagagtc aggggtctgt attttcctgc 60

tggtggctcc agttcaggaa cagtaaaccc tgctccgaat attgcctctc acatctcgtc 120

aatctccgcg aggactgggg accctgtghc gaacatggag aacatcacat caggattcct 180

aggacccctg ctcgtgttac aggcggggtt tttcttgttg acaagaatcc tcacaatacc 240

gcagagtcta gactcgtggt ggacttctct caattttcta gggggatcac ccgtgtgtct 300

tggccaaaat tcgcagtccc caacctccaa tcactcacca acctcctgtc ctccaatttg 360

tcctggttat cgctggatgt gtctgcggcg ttttatcata ttcctcttca tcctgctgct 420

atgcctcatc ttcttattgg ttcttctgga ttatcaaggt atgttgcccg tttgtcctct 480

aattccagga tcaacaacaa ccagtacggg accatgcaaa acctgcacga ctcctgctca 540

aggcaactct atgtttccct catgttgctg tacaaaacct acggatggaa attgcacctg 600

tattcccatc ccatcgtctt gggctttcgc aaaataccta tgggagtggg cctcagtccg 660

tttctcttgg ctcagtttac tagtgccatt tgttcagtgg ttcgtagggc tttcccccac 720

tgtttggctt tcagctatat ggatgatgtg gtattggggg ccaagtctgt acagcatcgt 780

gagtcccttt ataccgctgt taccaatttt cttttgtctc tgggtataca tttaaaccct 840

aacaaaacaa aaagatgggg ttattcccta aacttcatgg gttatgtaat tggaagttgg 900

ggaacattgc cacaggatca tattgtacaa aaaatcaaac actgttttag aaaacttcct 960

gttaacaggc ctattgattg gaaagtatgt caaagaattg tgggtctttt gggctttgct 1020

gctccattta cacaatgtgg atatcctgcc ttaatgcctt tgtatgcatg tatacaagct 1080

aaacaggctt tcactttctc gccaacttac aaggcctttc taagtaaaca gtacatgaac 1140

ctttaccccg ttgctcggca acggcctggt ctgtgccaag tgtttgctga cgcaaccccc 1200

actggctggg gcttggccat aggccatcag cgcatgcgtg gaacctttgt ggctcctctg 1260

ccgatccata ctgcggaact cctagccgct tgttttgctc gcagccggtc tggagcaaav 1320

ctcatcggaa ctgacaattc tgtcgtcctc tcgcggaaat atacatcgtt tccatggctg 1380

ctaggctgta ctgccaactg gatccttcgc gggacgtcct ttgtttacgt cccgtcggcg 1440

ctgaatcccg cggacgaccc ctctcggggc cgcttgggac tctatcgtcc ccttctccgt 1500

ctgccgttcc agccgaccac ggggcgcacc tctctttacg cggtctcccc gtctgtgcct 1560

tctcatctgc cggtccgtgt gcacttcgct tcacctctgc acgttgcatg gagaccaccg 1620

tgaacgccca tcagatcctg cccaaggtct tacataagag gactcttgga ctcccagcaa 1680

tgtcaacgac cgaccttgag gcctacttca aagactgtgt gtttaaggac tgggaggagc 1740

tgggggagga gattaggtta aagdtctttg tattaggagg ctgtaggcat aaattggtct 1800

gcgcaccagc accatgcaac tttttcacct ctgcctaatc atctcttgta catgtcccac 1860

tgttcaagcc tccaagctgt gccttgggtg gctttggggc atggacattg acccttataa 1920

agaatttgga gctactgtgg agttactctc gtttttgcct tctgacttct ttccttccgt 1980

cagagatctc ctagacaccg cctcagctct gtatcgggaa gccttagagt ctcctgagca 2040

ttgctcacct caccatactg cactcaggca agccattctc tgctgggggg aattgatgac 2100

tctagctacc tgggtgggta ataatttgga agatccagca tccagggatc tagtagtcaa 2160

ttatgttaat actaacatgg gyytaaagat caggcaacta ttgtggtttc atatwtcttg 2220

ccttactttt ggaagagara ctgtacttga atatttggtc tctttcggag tgtggattcg 2280

cactcctcca gcctatagac caccaaatgc ccctatctta tcaacacttc cggaaactac 2340

tgttgttaga cgacgggacc gaggcaggtc ccctagaaga agaactccct cgcctcgcag 2400

acgcagatct caatcgccgc gtcgcagaag atctcaatct cgggaatctc aatgttagta 2460

ttccttggac tcataaggtg ggaaacttta ctgggcttta ttcctctaca gtacctatct 2520

ttaatcctga atggcaaact ccttcctttc ctaagattca tttacaagag gacattatta 2580

ataggtgtca acaatttgtg ggccctctca ctgtaaatga aaagagaaga ttgaaattaa 2640

ttatgcctgc tagattctat cctacccaca ctaaatattt gcccttagac aaaggaatta 2700

aaccttatta tccagatcag gtagttaatc attacttcca aaccagacat tatttacata 2760

ctctttggaa ggctggtatt ctatataaga gggaaaccac acgtagcgca tcattttgcg 2820

ggtcaccata ttcttgggaa caagagctac agcatgggag gttggtcatc aaaacctcgc 2880

aaaggcatgg ggacgaatct ttctgttccc aaccctctgg gattctttcc cgatcatcag 2940

ttggaccctg cattcggagc caactcaaac aatccagatt gggacttcaa ccccatcaag 3000

gaccactggc cagmagccaa ccaggtagga gtgggagcat tcgggccagg gttcacccct 3060

ccacacggmg gtvttttggg gtggagccct caggctcagg gcatattgac cacagtgyca 3120

acaattcctc ctcctgcctc caccaatcgg cagtcaggaa ggcagcctac tcccatctct 3180

ccacctctaa gagacagtca tcctcaggcc atgcagtgga a 3221

<210>  3
<211>  3215
<212>  DNA
<213>  Artificial Sequence

<220>
<223>  HBV genotype B consensus sequence

<220>
<221>  misc_feature
<222>  (1632)..(1632)
<223>  n = a, c, t or g


<220>
<221>  misc_feature
<222>  (2699)..(2699)
<223>  n = a, c, t or g


<400>  3
ctccaccact ttccaccaaa ctcttcaaga tcccagagtc agggccctgt actttcctgc      60

tggtggctcc agttcaggaa cagtgagccc tgctcagaat actgtctctg ccatatcgtc     120

aatcttatcg aagactgggg accctgtacc gaacatggag aacatcgcat caggactcct     180

aggacccctg ctcgtgttac aggcggggtt tttcttgttg acaaaaatcc tcacaatacc     240

acagagtcta gactcgtggt ggacttctct caattttcta gggggaacac ccgtgtgtct     300

tggccaaaat tcgcagtccc aaatctccag tcactcacca acctgttgtc ctccaatttg     360

tcctggttat cgctggatgt gtctgcggcg ttttatcatc ttcctctgca tcctgctgct     420

atgcctcatc ttcttgttgg ttcttctgga ctatcaaggt atgttgcccg tttgtcctct     480

aattccagga tcatcaacaa ccagcaccgg accatgcaaa acctgcacaa ctcctgctca     540

aggaacctct atgtttccct catgttgctg tacaaaacct acggacggaa actgcacctg     600

tattcccatc ccatcatctt gggctttcgc aaaataccta tgggagtggg cctcagtccg     660

tttctcttgg ctcagtttac tagtgccatt tgttcagtgg ttcgtagggc tttcccccac     720

tgtctggctt tcagttatat ggatgatgtg gttttggggg ccaagtctgt acaacatctt     780

gagtcccttt atgccgctgt taccaatttt cttttgtctt tgggtataca tttaaaccct     840

cacaaaacaa aaagatgggg atattccctt aacttcatgg gatatgtaat tgggagttgg     900

ggcacattgc cacaggaaca tattgtacaa aaaatcaaaa tgtgttttag gaaacttcct     960

gtaaacaggc ctattgattg gaaagtatgt caacgaattg tgggtctttt ggggtttgcc    1020

gcccctttca cgcaatgtgg atatcctgct ttaatgcctt tatatgcatg tatacaagca    1080

aaacaggctt ttactttctc gccaacttac aaggcctttc taagtaaaca gtatctgaac    1140

ctttaccccg ttgctcggca acggcctggt ctgtgccaag tgtttgctga cgcaaccccc    1200

actggttggg gcttggccat aggccatcag cgcatgcgtg gaacctttgt gtctcctctg    1260

ccgatccata ctgcggaact cctagccgct tgttttgctc gcagcaggtc tggggcaaaa 1320

ctcatcggga ctgacaattc tgtcgtgctc tcccgcaagt atacatcatt tccatggctg 1380

ctaggctgtg ctgccaactg gatcctgcgc gggacgtcct ttgtttacgt cccgtcggcg 1440

ctgaatcccg cggacgaccc ctcccggggc cgcttggggc tctaccgccc gcttctccgc 1500

ctgttgtacc gaccgaccac ggggcgcacc tctctttacg cggactcccc gtctgtgcct 1560

tctcatctgc cggaccgtgt gcacttcgct tcacctctgc acgtcgcatg gagaccaccg 1620

tgaacgccca cnggaacctg cccaaggtct tgcataagag gactcttgga ctttcagcaa 1680

tgtcaacgac cgaccttgag gcatacttca aagactgtgt gtttactgag tgggaggagt 1740

tgggggagga gattaggtta aaggtctttg tactaggagg ctgtaggcat aaattggtgt 1800

gttcaccagc accatgcaac tttttcacct ctgcctaatc atctcatgtt catgtcctac 1860

tgttcaagcc tccaagctgt gccttgggtg gctttggggc atggacattg acccgtataa 1920

agaatttgga gcttctgtgg agttactctc ttttttgcct tctgacttct ttccttctat 1980

tcgagatctc ctcgacaccg cctctgctct gtatcgggag gccttagagt ctccggaaca 2040

ttgttcacct caccatacgg cactcaggca agctattctg tgttggggtg agttaatgaa 2100

tctagccacc tgggtgggaa gtaatttgga agatccagca tccagggaat tagtagtcag 2160

ctatgtcaac gttaatatgg gcctaaaaat cagacaacta ttgtggtttc acatttcctg 2220

tcttactttt gggagagaaa ctgttcttga atatttggtg tcttttggag tgtggattcg 2280

cactcctcct gcatatagac caccaaatgc ccctatctta tcaacacttc cggaaactac 2340

tgttgttaga cgaagaggca ggtcccctag aagaagaact ccctcgcctc gcagacgaag 2400

gtctcaatcg ccgcgtcgca gaagatctca atctcgggaa tctcaatgtt agtattcctt 2460

ggacacataa ggtgggaaac tttacggggc tttattcttc tacggtacct tgctttaatc 2520

ctaaatggca aactccttct tttcctgaca ttcatttgca ggaggacatt gttgatagat 2580

gtaagcaatt tgtggggccc cttacagtaa atgaaaacag gagactaaaa ttaattatgc 2640

ctgctaggtt ttatcccaat gttactaaat atttgccctt agataaaggg atcaaaccnt 2700

attatccaga gcatgtagtt aatcattact tccagacgag acattattta cacactcttt 2760

ggaaggcggg batcttatat aaaagagagt ccacacgtag cgcctcattt tgcgggtcac 2820

catattcttg ggaacaagat ctacagcatg ggaggttggt cttccaaacc tcgaaaaggc 2880

atggggacaa atctttctgt ccccaatccc ctgggattct tccccgatca tcagttggac 2940

cctgcattca aagccaactc agaaaatcca gattgggacc tcaacccgca caaggacaac 3000

tggccggacg ccaacaaggt gggagtggga gcattcgggc cagggttcac ccctccccat 3060

gggggactgt tggggtggag ccctcaggct cagggcctac tcacaactgt gccagcagct 3120

cctcctcctg cctccaccaa tcggcagtca ggaaggcagc ctactccctt atctccacct 3180

ctaagggaca ctcatcctca ggccatgcag tggaa 3215

<210> 4
<211> 3213
<212> DNA
<213> Artificial Sequence

<220>
<223> HBC genotype C consensus sequence

<220>
<221> misc_feature
<222> (1477)..(1477)
<223> n = a, c, t or g

<400> 4
   ctccacaaca ttccaccaag ctctgctaga tcccagagtg aggggcctat aytttcctgc 60
   tggtggctcc agttccggaa cagtaaaccc tgttccgact actgcctcac ccatatcgtc 120
   aatcttctcg aggactgggg accctgcacc gaacatggag aacacaacat caggattcct 180
   aggacccctg ctcgtgttac aggcggggtt tttcttgttg acaagaatcc tcacaatacc 240
   acagagtcta gactcgtggt ggacttctct caattttcta gggggagcac ccacgtgtcc 300
   tggccaaaat tcgcagtccc caacctccaa tcactcacca acctcttgtc ctccaatttg 360
   tcctggctat cgctggatgt gtctgcggcg ttttatcata ttcctcttca tcctgctgct 420
   atgcctcatc ttcttgttgg ttcttctgga ctaccaaggt atgttgcccg tttgtcctct 480
   acttccagga acatcaacta ccagcacggg accatgcaag acctgcacga ttcctgctca 540
   aggaacctct atgtttccct cttgttgctg tacaaaacct tcggacggaa actgcacttg 600
   tattcccatc ccatcatcct gggctttcgc aagattccta tgggagtggg cctcagtccg 660
   tttctcctgg ctcagtttac tagtgccatt tgttcagtgg ttcgtagggc tttcccccac 720
   tgtttggctt tcagttatat ggatgatgtg gtattggggg ccaagtctgt acaacatctt 780
   gagtcccttt ttacctctat taccaatttt cttttgtctt tgggtataca tttgaaccct 840
   aataaaacca aacgttgggg ctactccctt aacttcatgg gatatgtaat tggaagttgg 900
   ggtactttac cacaggaaca tattgtacta aaaatcaagc aatgttttcg aaaactgcct 960
   gtaaatagac ctattgattg gaaagtatgt caaagaattg tgggtctttt gggctttgct 1020
   gcccctttta cacaatgtgg ctatcctgcc ttaatgcctt tatatgcatg tatacaatct 1080
   aagcaggctt tcactttctc gccaacttac aaggcctttc tgtgtaaaca atatctgaac 1140
   ctttaccccg ttgcccggca acggtcaggt ctctgccaag tgtttgctga cgcaaccccc 1200
   actggatggg gcttggccat aggccatcgg cgcatgcgtg gaacctttgt ggctcctctg 1260
   ccgatccata ctgcggaact cctagcagct tgttttgctc gcagccggtc tggagcgaaa 1320
   cttatcggac gacaactctg ttgtcctctc tcggaaatac acctcctttc catggctgct 1380
   agggtgtgct gccaactgga tcctgcgcgg gacgtccttt gtctacgtcc cgtcggcgct 1440
   gaatcccgcg gacgacccgt ctcggggccg tttgggnctc taccgtcccc ttcttcatct 1500
   gccgttccgg ccgaccacgg ggcgcacctc tctttacgcg gtctccccgt ctgtgccttc 1560
   tcatctgccg gaccgtgtgc acttcgcttc acctctgcac gtcgcatgga gaccaccgtg 1620
   aacgcccacc aggtcttgcc caaggtctta cataagagga ctcttggact ctcagcaatg 1680
   tcaacgaccg accttgaggc atacttcaaa gactgtttgt ttaaagactg ggaggagttg 1740
   ggggaggaga ttaggttaat gatctttgta ctaggaggct gtaggcataa attggtctgt 1800
   tcaccagcac catgcaactt tttcacctct gcctaatcat ctcatgttca tgtcctactg 1860
   ttcaagcctc caagctgtgc cttgggtggc tttggggcat ggacattgac ccgtataaag 1920
   aatttggagc ttctgtggag ttactctctt ttttgccttc tgacttcttt ccttctattc 1980
   gagatctcct cgacaccgcc tctgctctgt atcgggaggc cttagagtct ccggaacatt 2040
   gttcacctca ccatacagca ctcaggcaag ctattctgtg ttggggtgag ttgatgaatc 2100
   tggccacctg ggtgggaagt aatttggaag acccagcatc cagggaatta gtagtcagct 2160
   atgtcaatgt taatatgggc ctaaaaatca gacaactatt gtggtttcac atttcctgtc 2220
   ttacttttgg aagagaaact gttcttgagt atttggtgtc ttttggagtg tggattcgca 2280
   ctcctcccgc ttacagacca ccaaatgccc ctatcttatc aacacttccg gaaactactg 2340
   ttgttagacg acgaggcagg tcccctagaa gaagaactcc ctcgcctcgc agacgaaggt 2400
   ctcaatcgcc gcgtcgcaga agatctcaat ctcgggaatc tcaatgttag tatcccttgg 2460
   actcataagg tgggaaactt tactgggctt tattcttcta ctgtacctgt ctttaatcct 2520
   gagtggcaaa ctccctcctt tcctcacatt catttacagg aggacattat taatagatgt 2580
   caacaatatg tgggccctct tacagttaat gaaaaaagga gattaaaatt aattatgcct 2640
   gctaggttct atcctaacct taccaaatat ttgcccttgg ataaaggcat taaaccttat 2700
   tatcctgaac atgcagttaa tcattacttc aaaactaggc attatttaca tactctgtgg 2760
   aaggctggca ttctatataa gagagaaact acacgcagcg cctcattttg tgggtcacca 2820
   tattcttggg aacaagagct acagcatggg aggttggtct tccaaacctc gacaaggcat 2880
   ggggacgaat ctttctgttc ccaatcctct gggattcttt cccgatcacc agttggaccc 2940
   tgcgttcgga gccaactcaa acaatccaga ttgggacttc aaccccaaca aggatcactg 3000
   gccagaggca aatcaggtag gagcgggagc attcgggcca gggttcaccc caccacacgg 3060
   cggtcttttg ggtggagccc tcaggctcag gggcatattg acaacagtgc cagcagcacc 3120
   tcctcctgcc tccaccaatc ggcagtcagg aagacagcct actcccatct ctccacctct 3180
   aagagacagt catcctcagg ccatgcagtg gaa 3213

<210> 5
<211> 3182
<212> DNA
<213> Artificial Sequence

<220>
<223> HBV genotype D consensus sequence

<400> 5
ctccacaacc ttccaccaaa ctctgcaaga tcccagagtg agaggcctgt atttccctgc 60

tggtggctcc agttcaggaa cagtaaaccc tgttccgact actgcctctc ccatatcgtc 120

aatcttctcg aggattgggg accctgcgct gaacatggag aacatcacat caggattcct 180

aggacccctg ctcgtgttac aggcggggtt tttcttgttg acaagaatcc tcacaatacc 240

gcagagtcta gactcgtggt ggacttctct caattttcta gggggaacta ccgtgtgtct 300

tggccaaaat tcgcagtccc caacctccaa tcactcacca acctcctgtc ctccaacttg 360

tcctggttat cgctggatgt gtctgcggcg ttttatcatc ttcctcttca tcctgctgct 420

atgcctcatc ttcttgttgg ttcttctgga ctatcaaggt atgttgcccg tttgtcctct 480

aattccagga tcttcaacca ccagcacggg accatgcaga acctgcacga ctcctgctca 540

aggaacctct atgtatccct cctgttgctg taccaaacct tcggacggaa attgcacctg 600

tattcccatc ccatcatcct gggctttcgg aaaattccta tgggagtggg cctcagcccg 660

tttctcctgg ctcagtttac tagtgccatt tgttcagtgg ttcgtagggc tttcccccac 720

tgtttggctt tcagttatat ggatgatgtg gtattggggg ccaagtctgt acagcatctt 780

gagtcccttt ttaccgctgt taccaatttt cttttgtctt tgggtataca tttaaaccct 840

aacaaaacaa aaagatgggg ttactcttta catttcatgg gctatgtcat tggatgttat 900

gggtcattgc cacaagatca catcatacag aaaatcaaag aatgttttag aaaacttcct 960

gttaacaggc ctattgattg gaaagtctgt caacgtattg tgggtctttt gggttttgct 1020

gcccctttta cacaatgtgg ttatcctgct ttaatgccct tgtatgcatg tattcaatct 1080

aagcaggctt tcactttctc gccaacttac aaggcctttc tgtgtaaaca atacctgaac 1140

ctttaccccg ttgcccggca acggccaggt ctgtgccaag tgtttgctga cgcaaccccc 1200

actggctggg gcttggtcat gggccatcag cgcatgcgtg gaacctttct ggctcctctg 1260

ccgatccata ctgcggaact cctagccgct tgttttgctc gcagcaggtc tggagcaaac 1320

attctcggga cggataactc tgttgttctc tcccgcaaat atacatcgtt tccatggctg 1380

ctaggctgtg ctgccaactg gatcctgcgc gggacgtcct ttgtttacgt cccgtcggcg 1440

ctgaatcccg cggacgaccc ttctcggggc cgcttgggac tctctcgtcc ccttctccgt 1500

ctgccgtttc gaccgaccac ggggcgcacc tctctttacg cggactcccc gtctgtgcct 1560

tctcatctgc cggaccgtgt gcacttcgct tcacctctgc acgtcgcatg gagaccaccg 1620

tgaacgccca ccaattcttg cccaaggtct tacataagag gactcttgga ctctctgtaa 1680

tgtcaacgac cgaccttgag gcatacttca aagactgttt gtttaaagac tgggaggagt 1740

tgggggagga gattagatta aaggtctttg tactaggagg ctgtaggcat aaattggtct 1800

gcgcaccagc accatgcaac tttttcacct ctgcctaatc atctcttgtt catgtcctac 1860

tgttcaagcc tccaagctgt gccttgggtg gctttggggc atggacattg atccttataa 1920

agaatttgga gctactgtgg agttactctc gtttttgcct tctgacttct ttccttcagt 1980

acgagatctt ctagataccg cctcagctct gtatcgggaa gccttagagt ctcctgagca 2040

ttgttcacct caccatactg cactcaggca agcaattctt tgctgggggg aactaatgac 2100

tctagctacc tgggtgggtg gtaatttgga agatccagca tccagggacc tagtagtcag 2160

ttatgtcaac actaatatgg gcctaaagtt caggcaacta ttgtggtttc acatttcttg 2220

tctcactttt ggaagagaaa cggtcataga gtatttggtg tctttcggag tgtggattcg 2280

cactcctcca gcttatagac caccaaatgc ccctatctta tcaacacttc cggagactac 2340

tgttgttaga cgacgaggca ggtcccctag aagaagaact ccctcgcctc gcagacgaag 2400

gtctcaatcg ccgcgtcgca gaagatctca atctcgggaa tctcaatgtt agtattcctt 2460

ggactcataa ggtgggaaac tttacggggc tttattcttc tactgtacct gtctttaacc 2520

ctcattggaa aacaccctct tttcctaata tacatttaca ccaagacatt atcaaaaaat 2580

gtgaacaatt tgtaggccca ctcacagtca atgagaaaag aagactgcaa ttgattatgc 2640

ctgctaggtt ttatccaaat gttaccaaat atttgccatt ggataagggt attaaacctt 2700

attatccaga acatctagtt aatcattact tccaaaccag acattattta cacactctat 2760

ggaaggcggg tatattatat aagagagaaa caacacatag cgcctcattt tgtgggtcac 2820

catattcttg ggaacaagag ctacagcatg gggcagaatc tttccaccag caatcctctg 2880

ggattctttc ccgaccacca gttggatcca gccttcagag caaacaccgc aaatccagat 2940

tgggacttca atcccaacaa ggacacctgg ccagacgcca acaaggtagg agctggagca 3000

ttcgggctgg gattcacccc accgcacgga ggccttttgg ggtggagccc tcaggctcag 3060

ggcatactac aaaccttgcc agcaaatccg cctcctgcct ctaccaatcg ccagtcagga 3120

aggcagccta ccccgctgtc tccacctttg agaaacactc atcctcaggc catgcagtgg 3180

aa 3182

<210> 6
<211> 3212
<212> DNA
<213> Artificial Sequence

<220>
<223> HBV genotype E consensus sequence

<400> 6
ttccacaaca ttccaccaag ctctgcagga tcccagagta agaggcctgt attttcctgc 60

tggtggctcc agttccggaa cagtgaaccc tgttccgact actgcctcac tcatctcgtc 120

aatcttctcg aggattgggg accctgcacc gaacatggaa agcatcacat caggattcct 180

aggacccctg ctcgtgttac aggcggggtt tttcttgttg acaaaaatcc tcacaatacc 240

gcagagtcta gactcgtggt ggacttctct caattttcta gggggagctc ccgtgtgtct 300

tggccaaaat tcgcagtccc caacctccaa tcactcacca acctcttgtc ctccaatttg 360

tcctggctat cgctggatgt gtctgcggcg ttttatcatc ttcctcttca tcctgctgct 420

atgcctcatc ttcttgttgg ttcttctgga ctatcaaggt atgttgcccg tttgtcctct 480

aattccagga tcatcaacca ccagtacggg accctgccga acctgcacga ctcttgctca 540

aggaacctct atgtttccct catgttgctg ttcaaaacct tcggacggaa attgcacttg 600

tattcccatc ccatcatcat gggctttcgg aaaattccta tgggagtggg cctcagcccg 660

tttctcctgg ctcagtttac tagtgccatt tgttcagtgg ttcgccgggc tttcccccac 720

tgtctggctt tcagttatat ggatgatgtg gtattggggg ccaagtctgt acaacatctt 780

gagtcccttt atacctctgt taccaatttt cttttgtctt tgggtataca tttaaatccc 840

aacaaaacaa aaagatgggg atattcccta aatttcatgg gttatgtaat tggaagttgg 900

gggtcattac cacaggaaca catcataaaa aaaatcaaag actgttttag aaaactccct 960

gttaaccggc ctattgattg gaaagtatgt caavgaattg tgggtctttt gggctttgct 1020

gcccctttta cacaatgtgg atatcctgct ttaatgcctc tgtatgcgtg tattcaatct 1080

aagcaggctt tcactttctc gccaacttac aaggcctttc tgtgtaaaca atacctgaac 1140

ctttaccccg ttgcccggca acggccaggt ctgtgccaag tgtttgctga tgcaaccccc 1200

actggctggg gcttggccat aggccatcag cgcatgcgcg gaacctttgt ggctcctctg 1260

ccgatccata ctgcggaact cctagccgct tgttttgctc gcagcaggtc tggagcaaaa 1320

cttatcggga cagataattc tgtcgttctc tcccggaaat atacatcctt tccatggctg 1380

ctaggctgtg ctgccaactg gatcctgcga gggacgtcct ttgtctacgt cccgtcagcg 1440

ctgaatcctg cggacgaccc gtctcggggt cgcttgggga tctatcgtcc ccttctccgt 1500

ctgccgttcc agccgaccac ggggcgcacc tctctttacg cggtctcccc gtctgtgcct 1560

tctcatctgc cggaccgtgt gcacttcgct tcacctctgc acgtcgcatg gagaccaccg 1620

tgaacgccca ccaaatcttg cccaaggtct tacataagag gactcttgga ctctctgcaa 1680

tgtcaacgac cgaccttgag gcatacttca aagactgttt gtttaaagac tgggaggagt 1740

tgggggagga gattagatta aaggtctttg tactaggagg ctgtaggcat aaattggtct 1800

gcgcaccagc accatgcaac tttttcacct ctgcctaatc atctcttgtt catgtcctac 1860

tgttcaagcc tccaagctgt gccttgggtg gctttggggc atggacattg acccttataa 1920

agaatttgga gctactgtgg agttactctc gtttttgcct tctgacttct ttccttcagt 1980

aagagatctt ctagataccg cctcagctct gtatcgggat gccttagaat ctcctgagca 2040

ttgttcaccg caccacactg cactcaggca agccattctt tgctgggggg aactaatgac 2100

tctagctacc tgggtgggtg taaatttgga agatccagca tccagggacc tagtagtcag 2160

ttatgtcaat actaatatgg gcctaaagtt caggcaatta ttgtggtttc acatttcttg 2220

tctcactttt ggaagagaaa ccgtcataga gtatttggtg tcttttggag tgtggattcg 2280

cactcctcca gcttatagac caccaaatgc ccctatctta tcaacacttc cggagaatac 2340

tgttgttaga cgaagaggca ggtcccctag aagaagaact ccctcgcctc gcagacgaag 2400

atctcaatcg ccgcgtcgca gaagatctca atctccagct tcccaatgtt agtattcctt 2460

ggactcacaa ggtgggaaat tttacggggc tttactcttc tactatacct gtctttaatc 2520

ctaactggaa aactccatct tttcctgata ttcatttgca ccaggacatt attaacaaat 2580

gtgaacaatt tgtaggtcct ctaacagtaa atgaaaaacg aagattaaac ttagtcatgc 2640

ctgctagatt ttttcccatc tctacgaaat atttgcccct agagaaaggt ataaaacctt 2700

attatccaga taatgtagtt aatcattact tccaaaccag acactattta cataccctat 2760

ggaaggcggg catcttatat aaaagagaaa ctacacgtag cgcctcattt tgtgggtcac 2820

cttattcttg ggaacaagag ctacatcatg ggctttcttt ggacggtccc tctcgaatgg 2880

gggaagaatc attccaccac caatcctctg ggattttttc ccgaccacca gttggatcca 2940

gcattcagag caaacaccag aaatccagat tgggaccaca atcccaacaa agaccactgg 3000

acagaagcca acaaggtagg agtgggagca ttcgggccgg ggttcactcc cccacacgga 3060

ggccttttgg ggtggagccc tcaggctcaa ggcatgctaa aaacattgcc agcagatccg 3120

cctcctgcct ccaccaatcg gcagtcagga aggcagccta ccccaatcac tccacctttg 3180

agagacactc atcctcaggc catgcagtgg aa 3212

<210> 7
<211> 3215
<212> DNA
<213> Artificial Sequence

<220>
<223> HBV genotype F consensus sequence

<220>
<221> misc_feature
<222> (951)..(951)
<223> n = a, c, t or g

<220>
<221> misc_feature
<222> (1020)..(1020)
<223> n = a, c, t or g

<220>
<221> misc_feature
<222> (2690)..(2690)
<223> n = a, c, t or g

<400> 7
ctcaacccag ttccaccagg ctctgttaga tccgagggta agggctctgt attttcctgc 60

tggtggctcc agttcaggga cacagaaccc tgctccgact attgcctctc tcacatcatc 120

aatcttctcg aagactgggg gccctgctat gaacatggag aacatcacat caggactcct 180

aggacccctg ctcgtgttac aggcggtgtg tttcttgttg acaaaaatcc tcacaatacc 240

acagagtcta gactcgtggt ggacttctct caattttcta gggggactac ccgggtgtcc 300

tggccaaaat tcgcagtccc caacctccaa tcacttacca acctcctgtc ctccaacttg 360

tcctggctat cgttggatgt gtctgcggcg ttttatcatc ttcctcttca tcctgctgct 420

atgcctcatc ttcttgttgg ttcttctgga ctatcaaggt atgttgcccg tttgtcctct 480

acttccagga tccacaacca ccagcacggg accatgcaaa acctgcacaa ctcttgctca 540

aggaacctct atgtttccct cctgttgctg ttccaaaccc tcggacggaa actgcacctg 600

tattcccatc ccatcatctt gggctttagg aaaataccta tgggagtggg cctcagcccg 660

tttctcctgg ctcagtttac tagtgcaatt tgttcagtgg tgcgtagggc tttcccccac 720

tgtctggctt ttagttatat ggatgatctg gtattggggg ccaaatctgt gcagcatctt 780

gagtcccttt ataccgctgt taccaatttt ttgttatctg tgggtatcca tttaaatach 840

tctaaaacaa aaagatgggg ttacaaccta catttcatgg gttatgttat tggtagttgg 900

ggaacgttac cccaagatca tattgtacac aaaatcaaag attgttttcg naaacttcct 960

gtaaatcgtc caattgattg gaaagtttgt caacgcattg tgggtctttt gggctttgcn 1020

gcccctttca cccaatgtgg ttatcctgct ctcatgcctt tgtatgcctg tattactgct 1080

aaacaggctt ttgtcttttc gccaacttac aaggcctttc tctgtaaaca atacatgaac 1140

ctttaccccg ttgctcggca acggccaggc ctgtgccaag tgtttgctga cgcaaccccc 1200

actggttggg gcttggccat tggccatcag cgcatgcgtg gaacctttgt ggctcctctg 1260

ccgatccata ctgcggaact ccttgcagct tgtttcgctc gcagccggtc tggagcgaac 1320

attatcggca cagacaactc tgttgtcctc tctaggaagt acacctcctt tccatggctg 1380

ctcggttgtg ctgccaactg gatcctgcgc gggacgtcct ttgtttacgt cccgtcggcg 1440

ctgaatcccg cggacgaccc ctcccggggt cgcttggggc tgtaccgccc ccttctycgt 1500

ctgccgttcc agccgacgac gggtcgcacc tctctttacg cggactcccc gtctgttcct 1560
tctcatctgc cggaccgtgt gcacttcgct tcacctctgc acgtcgcatg gagaccaccg 1620
tgaacgcccc ctggaatttg ccaacagtct tacataagag gactcttgga ctttcaggac 1680
ggtcaatgac ctggatcgaa gaatacatca aagactgtgt atttaaggac tgggaggagc 1740
tgggggagga gatcaggtta aaggtctttg tactaggagg ctgtaggcat aaattggtct 1800
gttcaccagc accatgcaac tttttcacct ctgcctaatc atcttttgtt catgtcccac 1860
tgttcaagcc tccaagctgt gccttgggtg gctttggggc atggacattg acccttataa 1920
agaatttgga gcttctgtgg aattgctctc ttttttgcct tctgatttct tcccgtctgt 1980
tcgggaccta ctcgacaccg cttcagccct ttaccgggat gctttagagt caccggaaca 2040
ttgcaccccc aatcataccg ctctcaggca agctattttg tgctggggtg agttaatgac 2100
tttggcttcc tgggtgggta ataatttgga agaccctgca gctagggatt tagtagttaa 2160
ttatgtcaac actaatatgg gcctaaaaat tagacaactg ttgtggtttc acatttcctg 2220
ccttactttt ggaagagaaa cagttcttga gtatttggtg tcctttggag tgtggattcg 2280
cactccacct gcttatagac caccaaatgc ccctatccta tccacacttc cggaaactac 2340
tgttgttaga cgacgaggca ggtcccctag aagaagaact ccctcgcctc gcagacgaag 2400
atctcaatcg ccgcgtcgca gaagatctca atctccagct tcccaatgtt agtattcctt 2460
ggactcataa ggtgggaaat tttacggggc tctactcttc tactgtacct gctttcaatc 2520
ctaactggtt aactccttct tttcctgata ttcatttaca tcaagatctg atatctaaat 2580
gtgaacaatt tgtaggcccd ctcactaaaa atgaattgag aagattaaaa ttggttatgc 2640
cagctagatt ttatcctaag gttaccaaat actttcctat ggagaaaggn attaaaccct 2700
attatcctga gcatgcagtt aatcattatt ttaaaacbag acattatttg catactttdt 2760
ggaaggcggg aattttatat aagagagaat ccacacgtag cgcctcattt tgtgggtcac 2820
catattcctg ggaacaagag ctacagcatg ggagcacctc tctcaacgac aagaaggggc 2880
atgggacaga atctttctgt gcccaatcct ctgggattct tgccagacca tcagctggat 2940
ccgctattca gggcaaattc cagcagtccc gactgggact tcaacacaaa caaggacagt 3000
tggccaatgg caaacaaggt aggagtggga ggctacggtc cagggttcac acccccacac 3060
ggtggcctgc tggggtggag ccctcaggca cagggtgttt tdacaacctt gccagcagat 3120
ccgcctcctg cttccaccaa tcggcggtcc gggagaaagc caaccccagt ctctccacct 3180
ctaagagaca cacatccaca ggccatgcag tggaa 3215

<210> 8
<211> 3248
<212> DNA

<213>  Artificial Sequence

<220>
<223>  HBV genotype F consensus sequence

<400>  8
ctctacagca ttccaccaag ctctacaaaa tcccaaagtc aggggcctgt attttcctgc      60

tggtggctcc agttcaggga tagtgaaccc tgttccgact attgcctctc acatctcgtc     120

aatcttctcc aggattgggg accctgcacc gaacatggag aacatcacat caggattcct     180

aggacccctg ctcgtgttac aggcggggtt tttcttgttg acaagaatcc tcacaatacc     240

gcagagtcta gactcgtggt ggacttctct caattttcta gggggagtgc ccgtgtgtcc     300

tggcctaaat tcgcagtccc caacctccaa tcactcacca atctcctgtc ctccaacttg     360

tcctggctat cgctggatgt gtctgcggcg ttttatcata ttcctcttca tcctgctgct     420

atgcctcatc ttcttgttgg ttcttctgga ctatcaaggt atgttgcccg tttgtcctct     480

gattccagga tcctcgacca ccagtacggg accctgcaaa acctgcacga ctcctgctca     540

aggcaactct atgtatccct catgttgctg tacaaaacct tcggacggaa attgcacctg     600

tattcccatc ccatcatctt gggctttcgc aaaataccta tgggagtggg cctcagtccg     660

tttctcttgg ctcagtttac tagtgccatt tgttcagtgg ttcgtagggc tttcccccac     720

tgtctggctt tcagctatat ggatgatgtg gtattggggg ccaaatctgt acaacatctt     780

gagtcccttt ataccgctgt taccaatttt cttttgtctt tgggtataca tctaaaccct     840

aacaaaacaa aaagatgggg ttattcctta aattttatgg gatatgtaat tggaagttgg     900

ggtactttgc cacaagaaca catcacacag aaaattaagc aatgttttcg gaaactccct     960

gttaacaggc caattgattg gaaagtctgt caacgaataa ctggtctgtt gggtttcgct    1020

gctcctttta cccaatgtgg ttaccctgcc ttaatgcctt tatatgcatg tatacaagct    1080

aagcaggctt ttactttctc gccaacttat aaggcctttc tctgtaaaca atacatgaac    1140

ctttaccccg ttgctaggca acggcccggt ctgtgccaag tgtttgctga cgcaaccccc    1200

actggttggg gcttggccat cggccatcag cgcatgcgtg gaacctttgt ggctcctctg    1260

ccgatccata ctgcggaact cctagctgct tgttttgctc gcagccggtc tggagcaaaa    1320

ctcattggga ctgacaattc tgtcgtcctt tctcggaaat atacatcctt tccatggctg    1380

ctaggctgtg ctgccaactg gatccttcgc gggacgtcct ttgtttacgt cccgtcagcg    1440

ctgaatccag cggacgaccc ctcccggggc cgtttggggc tctgtcgccc ccttctccgt    1500

ctgccgttcc tgccgaccac ggggcgcacc tctctttacg cggtctcccc gtctgttcct    1560

tctcatctgc cggaccgtgt gcacttcgct tcacctctgc acgttacatg gaaaccgcca    1620

tgaacacctc tcatcatctg ccaaggcagt tatataagag gactcttgga ctgtttgtta    1680

tgtcaacaac cggggtggag aaatacttca aggactgtgt ttttgctgag tgggaagaat    1740

taggcaatga gtccaggtta atgacctttg tattaggagg ctgtaggcat aaattggtct 1800

gcgcaccagc accatgtaac tttttcacct ctgcctaatc atctcttgtt catgtcctac 1860

tgttcaagcc tccaagctgt gccttgggtg gctttagggc atggatagaa caactttgcc 1920

atatggcctt tttggcttag acattgaccc ttataaagaa tttggagcta ctgtggagtt 1980

gctctcgttt ttgccttctg actttttccc gtctgttcgt gatcttctcg acaccgcttc 2040

agctttgtac cgggaatcct tagagtcctc tgatcattgt tcgcctcacc atacagcact 2100

caggcaagca atcctgtgct ggggtgagtt gatgactcta gctacctggg tgggtaataa 2160

tttggaagat ccagcatcca gagatttggt ggtcaattat gttaatacta atatgggttt 2220

aaaaatcagg caactattgt ggtttcacat ttcctgtctt acttttggga gagaaaccgt 2280

tcttgagtat ttggtgtctt ttggagtgtg gattcgcact cctcctgctt atagaccacc 2340

aaatgcccct atcctatcaa cacttccgga gactactgtt gttagacgaa gaggcaggtc 2400

ccctcgaaga agaactccct cgcctcgcag acgaagatct caatcgccgc gtcgcagaag 2460

atctgcatct ccagcttccc aatgttagta ttccttggac tcacaaggtg ggaaacttta 2520

cggggctgta ttcttctact atacctgtct ttaatcctga ttggcaaact ccttcttttc 2580

caaatatcca tttgcatcaa gacattataa ctaaatgtga acaatttgtg ggccctctca 2640

cagtaaatga gaaacgaaga ttaaaactag ttatgcctgc cagatttttc ccaaactcta 2700

ctaaatattt accattagac aaaggtatca aaccgtatta tccagaaaat gtagttaatc 2760

attacttcca gaccagacat tatttacata ccctttggaa ggcgggtatt ctatataaga 2820

gagaaacatc ccgtagcgct tcattttgtg ggtcaccata tacttgggaa caagatctac 2880

agcatggggc tttcttggac ggtccctctc gagtggggaa agaacctttc caccagcaat 2940

cctctaggat tccttcccga tcaccagttg gacccagcat tcagagcaaa taccaacaat 3000

ccagattggg acttcaatcc caaaaaggac ccttggccag aggccaacaa ggtaggagtt 3060

ggagcctatg gacccgggtt cacccctcca cacggaggcc ttttggggtg gagccctcag 3120

tctcagggca cactaacaac tttgccagca gatccgcctc ctgcctccac caatcgtcag 3180

tcagggaggc agcctactcc catctctcca ccactaagag acagtcatcc tcaggccatg 3240

cagtggaa 3248

<210> 9
<211> 3215
<212> DNA
<213> Artificial Sequence

<220>
<223> HBV genotype H consensus sequence

<400> 9

ctcaacacag ttccaccaag cactgttgga tccgagagta aggggtctgt attttcctgc 60
tggtggctcc agttcagaaa cacagaaccc tgttccgact attgcctctc tcacatcatc 120
aatcttctcg aagactgggg accctgctat gaacatggag aacatcacat caggactcct 180
aggacccctt ctcgtgttac aggcggtgtg tttcttgttg acaaaaatcc tcacaatacc 240
acagagtcta gactcgtggt ggacttctct caattttcta ggggtaccac ccgggtgtcc 300
tggccaaaat tcgcagtccc caatctccaa tcacttacca acctcctgtc ctccaacttg 360
tcctggctat cgttggatgt gtctgcggcg ttttatcatc ttcctcttca tcctgctgct 420
atgcctcatc ttcttgttgg ttcttctgga ctatcaaggt atgttgcccg tgtgtcctct 480
acttccagga tctacaacca ccagcacggg accctgcaaa acctgcacca ctcttgctca 540
aggaacctct atgtttccct cctgctgctg taccaaacct tcggacggaa attgcacctg 600
tattcccatc ccatcatctt gggctttcgg aaaataccta tgggagtggg cctcagcccg 660
tttctcttgg ctcagtttac tagtgcaatt tgttcagtgg tgcgtagggc tttcccccac 720
tgtctggctt ttagttatat ggatgatttg gtattggggg ccaaatctgt gcagcatctt 780
gagtcccttt ataccgctgt taccaatttt ttgttatctg tgggcatcca tttaaacaca 840
gctaaaacaa aatggtgggg ttattcctta cactttatgg gttatatcat tgggagttgg 900
gggacattgc ctcaggaaca tattgtgcaa aaaatcaaag attgctttcg caaacttccc 960
gttaatcgac ccattgattg gaaagtctgt caacgaattg tgggtctttt gggctttgca 1020
gcccctttta ctcaatgtgg ttatcctgct ctcatgccct tatatgcctg tattaccgct 1080
aaacaggctt ttgttttctc gccaacttac aaggcctttc tctgtaaaca atacatgaac 1140
ctttaccccg ttgctcggca acggccaggc ctttgccaag tgtttgctga cgcaaccccc 1200
actggctggg gcttggcgat tggccatcag cgcatgcgcg gaacctttgt ggctcctctg 1260
ccgatccata ctgcggaact cctagcagct tgtttcgctc gcagccggtc tggagcggac 1320
attatcggca ctgacaactc cgttgtcctg tctcggaagt acacctcctt cccatggctg 1380
ctaggctgtg ctgccaactg gatcctgcgc gggacgtcct ttgtttacgt cccgtcggcg 1440
ctgaatcctg cggacgaccc ctctcgtggt cgcctggggc tctgccgccc ccttctccgc 1500
cttccgttcc ggccgacgac gggtcgcacc tctctttacg cggactcccc gcctgtgcct 1560
tctcatctgc cggcccgtgt gcacttcgct tcacctctgc acgtcgcatg gagaccaccg 1620
tgaacgcccc tcaaagcttg ccaacaacct tacataagag gactcttgga ctttcgcccc 1680
ggtcaacgac ctggattgag gaatacatca aagactgtgt gtttaaggac tgggaggagt 1740
cgggggagga gttgaggtta aaggtctttg tattaggagg ctgtaggcat aaattggtct 1800
gttcaccagc accatgcaac tttttcacct ctgcctaatc atcttttgtt catgtcccac 1860
tgttcaagcc tccaagctgt gccttgggtg gctttggggc atggacattg acccttataa 1920

agaatttgga gcttctgtgg agttactctc atttttgcct tctgacttct tcccgtctgt 1980

ccgggaccta ctcgacaccg cttcagccct ctaccgagat gccttagaat cacccgaaca 2040

ttgcaccccc aaccatactg ctctcaggca agctattctg tgctggggtg agttaatgac 2100

tttggcttcc tgggtgggca ataatttaga ggatcctgcg gctagagatc tagtagttaa 2160

ttatgtcaac actaatatgg gcctaaaaat tagacaatta ctatggtttc atatttcctg 2220

ccttacattt ggaagagaaa ctgttcttga gtatttggtg tcttttggag tgtggattcg 2280

cactccacct gcttatagac caccaaatgc ccctatccta tcaacacttc cggagactac 2340

tgttgttaga caacgaggca gggcccctag aagaagaact ccctcgcctc gcagacgaag 2400

atctcaatca ccgcgtcgca gaagatctca atctccagct tcccaatgtt agtatccctt 2460

ggactcataa ggtgggaaac tttaccggtc tttactcctc tactgtacct gttttcaatc 2520

ctgattggtt aactccttct tttcctgaca ttcacctaca tcaagatttg atacaaaaat 2580

gtgaacaatt tgtaggccca ctcactaaaa atgaagtgag acgattgaaa ttaattatgc 2640

cagcaaggtt ttatcccaaa gttactaaat acttcccttt ggataaaggt attaaaccat 2700

attatccaga gaatgtggtt aatcattact tcaaaactag acactattta catactttgt 2760

ggaaggcagg aattctatat aagagagaat ccacacatag cgcctcattt tgtgggtcac 2820

catattcctg ggaacaagag ctacagcatg ggagcacctc tctcaacggc gagaaggggc 2880

atgggacaga atctttctgt gcccaatcct ctgggattct ttccagacca ccagttggat 2940

ccactattca gagcaaattc cagcagtccc gattgggact tcaacacaaa caaggacaat 3000

tggccaatgg caaacaaggt aggagtggga ggctttggtc cagggttcac acccccacac 3060

ggtggccttc tggggtggag ccctcaggca cagggcattc tgacaacctc gccaccagat 3120

ccacctcccg cttccaccaa tcggaggtca ggaaggaaac caaccccagt ctctccacct 3180

ctaaggacac cacatccaca ggccatgcag tggaa 3215

<210> 10
<211> 6
<212> DNA
<213> Artificial Sequence

<220>
<223> restriction site for BssH2

<400> 10
gcgcgc 6

<210> 11
<211> 42
<212> DNA
<213> Artificial Sequence

<220>
<223>  primer

<220>
<221>  misc_feature
<222>  (1)..(1)
<223>  n = (N)x wherein N is any nucleotide and x is an integer at least
       equal to 3


<400>  11
ngcgcgccag caccatgcaa ctttttcacc tctgcctaat ca                        42


<210>  12
<211>  29
<212>  DNA
<213>  Artificial Sequence

<220>
<223>  primer

<220>
<221>  misc_feature
<222>  (29)..(29)
<223>  n = (N)x wherein N is any nucleotide and x is an integer at least
       equal to 3


<400>  12
gtgaaaaagt tgcatggtgc tggcgcgcn                                       29


<210>  13
<211>  6
<212>  DNA
<213>  Artificial Sequence

<220>
<223>  restriction site for Cla I

<400>  13
atcgat                                                                 6


<210>  14
<211>  33
<212>  DNA
<213>  Artificial Sequence

<220>
<223>  primer

<220>
<221>  misc_feature
<222>  (1)..(1)
<223>  n = (N)x wherein N is any nucleotide and x is an integer at least
       equal to 3


<400>  14
natcgatgca actttttcac ctctgcctaa tca                                  33

<210>  15
<211>  20
<212>  DNA
<213>  Artificial Sequence

<220>
<223>  primer

<220>
<221>  misc_feature
<222>  (20)..(20)
<223>  n = (N)x wherein N is any nucleotide and x is an integer at least
        equal to 3

<400>  15
gtgaaaaagt tgcatcgatn                                                    20

<210>  16
<211>  8
<212>  DNA
<213>  Artificial Sequence

<220>
<223>  example of "n" for SEQ ID No.15

<400>  16
ctggtgcg                                                                  8

<210>  17
<211>  6
<212>  DNA
<213>  Artificial Sequence

<220>
<223>  restriction site for Mlu I

<400>  17
acgcgt                                                                    6

<210>  18
<211>  38
<212>  DNA
<213>  Artificial Sequence

<220>
<223>  primer

<220>
<221>  misc_feature
<222>  (1)..(1)
<223>  n = (N)x wherein N is any nucleotide and x is an integer at least
        equal to 3

<400>  18
nacgcgtacc atgcaacttt ttcacctctg cctaatca                                38

<210>  19
<211>  25
<212>  DNA
<213>  Artificial Sequence

<220>
<223>  primer

<220>
<221>  misc_feature
<222>  (25)..(25)
<223>  n = (N)x wherein N is any nucleotide and x is an integer at least
        equal to 3


<400>  19
gtgaaaaagt tgcatggtac gcgtn                                              25


<210>  20
<211>  6
<212>  DNA
<213>  Artificial Sequence

<220>
<223>  example of "n" for SEQ ID No.19

<400>  20
cgcaga                                                                    6


<210>  21
<211>  6
<212>  DNA
<213>  Artificial Sequence

<220>
<223>  restriction site for Pvu I

<400>  21
cgatcg                                                                    6


<210>  22
<211>  37
<212>  DNA
<213>  Artificial Sequence

<220>
<223>  primer

<220>
<221>  misc_feature
<222>  (1)..(1)
<223>  n = (N)x wherein N is any nucleotide and x is an integer at least
        equal to 3


<400>  22
ncgatcgcca tgcaactttt tcacctctgc ctaatca                                 37


<210>  23

<211>  24
<212>  DNA
<213>  Artificial Sequence

<220>
<223>  primer

<220>
<221>  misc_feature
<222>  (24)..(24)
<223>  n = (N)x wherein N is any nucleotide and x is an integer at least
        equal to 3


<400>  23
gtgaaaaagt tgcatggcga tcgn                                              24


<210>  24
<211>  7
<212>  DNA
<213>  Artificial Sequence

<220>
<223>  Example of "n" for SEQ ID No.23

<400>  24
gcgcaga                                                                  7


<210>  25
<211>  6
<212>  DNA
<213>  Artificial Sequence

<220>
<223>  restriction site for Rca I

<400>  25
tcatga                                                                   6


<210>  26
<211>  37
<212>  DNA
<213>  Artificial Sequence

<220>
<223>  primer

<220>
<221>  misc_feature
<222>  (1)..(1)
<223>  n = (N)x wherein N is any nucleotide and x is an integer at least
        equal to 3


<400>  26
ntcatgacca tgcaactttt tcacctctgc ctaatca                               37


<210>  27
<211>  24

<212>  DNA
<213>  Artificial Sequence

<220>
<223>  primer

<220>
<221>  misc_feature
<222>  (24)..(24)
<223>  n = (N)x wherein N is any nucleotide and x is an integer at least
       equal to 3


<400>  27
gtgaaaaagt tgcatggtca tgan                                              24


<210>  28
<211>  8
<212>  DNA
<213>  Artificial Sequence

<220>
<223>  example of "n" for SEQ ID No.27

<400>  28
tgcgcaga                                                                 8


<210>  29
<211>  6
<212>  DNA
<213>  Artificial Sequence

<220>
<223>  restriction site for Nhe I

<400>  29
gctagc                                                                   6


<210>  30
<211>  39
<212>  DNA
<213>  Artificial Sequence

<220>
<223>  primer

<220>
<221>  misc_feature
<222>  (1)..(1)
<223>  n = (N)x wherein N is any nucleotide and x is an integer at least
       equal to 3


<400>  30
ngctagcacc atgcaacttt ttcacctctg cctaatcat                              39


<210>  31
<211>  25
<212>  DNA

<213>  Artificial Sequence

<220>
<223>  primer

<220>
<221>  misc_feature
<222>  (25)..(25)
<223>  n = (N)x wherein N is any nucleotide and x is an integer at least
       equal to 3


<400>  31
gtgaaaaagt tgcatggtgc tagcn                                          25


<210>  32
<211>  4
<212>  DNA
<213>  Artificial Sequence

<220>
<223>  example of "n" for SEQ ID No.31

<400>  32
gcgc                                                                  4


<210>  33
<211>  8
<212>  DNA
<213>  Artificial Sequence

<220>
<223>  restriction site for Not I

<400>  33
gcggccgc                                                              8


<210>  34
<211>  39
<212>  DNA
<213>  Artificial Sequence

<220>
<223>  primer

<220>
<221>  misc_feature
<222>  (1)..(1)
<223>  n = (N)x wherein N is any nucleotide and x is an integer at least
       equal to 3


<400>  34
ngcggccgca ccatgcaact ttttcacctc tgcctaatc                           39


<210>  35
<211>  27
<212>  DNA
<213>  Artificial Sequence

<220>
<223>  primer

<220>
<221>  misc_feature
<222>  (27)..(27)
<223>  n = (N)x wherein N is any nucleotide and x is an integer at least
       equal to 3


<400>  35
gtgaaaaagt tgcatggtgc ggccgcn                                          27


<210>  36
<211>  4
<212>  DNA
<213>  Artificial Sequence

<220>
<223>  example of "n" for SEQ ID No.35

<400>  36
gcag                                                                    4


<210>  37
<211>  6
<212>  DNA
<213>  Artificial Sequence

<220>
<223>  restriction site for Sun I (BsiW I)

<400>  37
cgtacg                                                                  6


<210>  38
<211>  42
<212>  DNA
<213>  Artificial Sequence

<220>
<223>  primer

<220>
<221>  misc_feature
<222>  (1)..(1)
<223>  n = (N)x wherein N is any nucleotide and x is an integer at least
       equal to 3


<400>  38
ncgtacgagc accatgcaac tttttcacct ctgcctaatc at                         42


<210>  39
<211>  28
<212>  DNA
<213>  Artificial Sequence

<220>
<223>  primer

<220>
<221>  misc_feature
<222>  (28)..(28)
<223>  n = (N)x wherein N is any nucleotide and x is an integer at least
       equal to 3


<400>  39
gtgaaaaagt tgcatggtgc tcgtacgn                                          28


<210>  40
<211>  4
<212>  DNA
<213>  Artificial Sequence

<220>
<223>  example of "n" for SEQ ID No.39

<400>  40
caga                                                                     4


<210>  41
<211>  6
<212>  DNA
<213>  Artificial Sequence

<220>
<223>  restriction site for Nar I

<400>  41
ggcgcc                                                                   6


<210>  42
<211>  37
<212>  DNA
<213>  Artificial Sequence

<220>
<223>  primer

<220>
<221>  misc_feature
<222>  (1)..(1)
<223>  n = (N)x wherein N is any nucleotide and x is an integer at least
       equal to 3


<400>  42
nggcgcccat gcaacttttt cacctctgcc taatcat                                37


<210>  43
<211>  22
<212>  DNA
<213>  Artificial Sequence

<220>

<223>  primer

<220>
<221>  misc_feature
<222>  (22)..(22)
<223>  n = (N)x wherein N is any nucleotide and x is an integer at least
       equal to 3


<400>  43
gtgaaaaagt tgcatggcgc cn                                              22


<210>  44
<211>  38
<212>  DNA
<213>  Artificial Sequence

<220>
<223>  primer

<400>  44
gggggcgcca tgcaactttt tcacctctgc ctaatcat                             38


<210>  45
<211>  27
<212>  DNA
<213>  Artificial Sequence

<220>
<223>  primer

<400>  45
gtgaaaaagt tgcatggcgc cggtgcg                                         27


<210>  46
<211>  19
<212>  DNA
<213>  Artificial Sequence

<220>
<223>  primer

<400>  46
tcgtggtgga cttctctca                                                  19


<210>  47
<211>  18
<212>  DNA
<213>  Artificial Sequence

<220>
<223>  primer

<400>  47
aaacgccgca gacacatc                                                   18


<210>  48
<211>  18

<212>  DNA
<213>  Artificial Sequence

<220>
<223>  primer

<400>  48
gaggcatagc agcaggat                                                    18


<210>  49
<211>  19
<212>  DNA
<213>  Artificial Sequence

<220>
<223>  primer

<400>  49
tggatgtgtc tgcggcgtt                                                   19


<210>  50
<211>  18
<212>  DNA
<213>  Artificial Sequence

<220>
<223>  primer

<400>  50
ggacaaacgg gcaacata                                                    18


<210>  51
<211>  18
<212>  DNA
<213>  Artificial Sequence

<220>
<223>  primer

<400>  51
gctgacgcaa cccccact                                                    18


<210>  52
<211>  18
<212>  DNA
<213>  Artificial Sequence

<220>
<223>  primer

<400>  52
aggagttccg cagtatgg                                                    18


22